(19) Europäisches Patentamt
European Patent Office
Office européen des brevets

(11) **EP 4 295 834 A1**

(12) **EUROPEAN PATENT APPLICATION**
published in accordance with Art. 153(4) EPC

(43) Date of publication:
**27.12.2023 Bulletin 2023/52**

(51) International Patent Classification (IPC):
**A61K 8/73** (2006.01)    **A61Q 5/00** (2006.01)
**A61Q 5/04** (2006.01)    **A61Q 5/06** (2006.01)

(21) Application number: **22763339.3**

(52) Cooperative Patent Classification (CPC):
**A61K 8/73; A61Q 5/00; A61Q 5/04; A61Q 5/06**

(22) Date of filing: **02.03.2022**

(86) International application number:
**PCT/JP2022/008915**

(87) International publication number:
**WO 2022/186286 (09.09.2022 Gazette 2022/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.03.2021 JP 2021035865**
**12.11.2021 JP 2021184640**

(71) Applicant: Hayashibara Co., Ltd.
**Okayama-shi, Okayama 7028006 (JP)**

(72) Inventors:
• **CHIKATOMO Ami**
**Okayama-shi Okayama 702-8006 (JP)**
• **SODA Kaho**
**Okayama-shi Okayama 702-8006 (JP)**
• **TANIGUCHI Mutsuko**
**Okayama-shi Okayama 702-8006 (JP)**
• **MIYAKE Masaki**
**Okayama-shi Okayama 702-8006 (JP)**
• **IWAKI Kanso**
**Okayama-shi Okayama 702-8006 (JP)**

(74) Representative: **Page White Farrer**
**Bedford House**
**21a John Street**
**London WC1N 2BF (GB)**

(54) **HAIR SETTING POWER ENHANCER**

(57) An object of the present invention is to provide a novel hair-setting composition having sufficient hair-setting ability, and a means to obtain such a hair-setting composition. The present invention attains the above object by providing an agent for enhancing a hair-setting ability comprising an isomaltooligosaccharide as an active ingredient, and a hair-setting composition comprising the agent.

EP 4 295 834 A1

**Description**

**Technical Field**

This invention relates to an agent for enhancing a hair-setting ability, a hair-setting composition comprising the agent, and a method for enhancing a hair-setting ability using the hair-setting composition. In more detail, this invention relates to an agent for enhancing a hair-setting ability comprising an isomaltooligosaccharide as an active ingredient and a hair-setting composition comprising the agent.

**Background Art**

Conventionally, as a hair-setting agent for setting hair, an agent prepared by dissolving a polymer with film-forming property in a solvent, such as water, lower alcohols, or mixture thereof has been commonly used. Polymers with film-forming property shows the effect of holding hair by adhering to the hair surface and fixing the hairs adhered to one another. The conventionally used film-forming polymers have the drawback that they became sticky during the process of from applying the polymers to hairs, dry finishing and the stickiness poses a restriction on freedom of hair styling. In order to overcome these drawbacks, attempts have been made to improve usability of film-forming polymers by adding thereto oils and fats, surfactants, alcohols, etc. for cosmetic uses, but a product having a hairholding ability and a feeling of use both at a satisfactory level has not been obtained.

In order to improve the hair-setting ability and feeling of use of conventional hair-setting compositions, methods involving formulating specific polymers and ingredients other than polymers therein or adjusting pH of the compositions to specific values have been proposed (For example, see Patent Literature 1.). A method involving formulating a sugar alcohol as an ingredient other than polymers (For example, see Patent Literature 2), and a method involving formulating a saccharide derivative of $\alpha$, $\alpha$-trehalose as an ingredient other than polymers (For example, see Patent Literature 3) have been proposed. However, improvement of hair-setting compositions by these methods was still insufficient in terms of elimination of stickiness in the process of from application to drying and feeling of use, such as smoothness. Even if they have managed to have good feeling of use, they were not fully satisfactory from a comprehensive point of view, since there were drawbacks that a sufficient setting ability cannot be maintained, resulting in a mess of hairstyle over time, and that unfavorable appearances, such as flaking (appearance of powders on the hair surface), were observed upon re-styling.

Meanwhile, there has been an increasing desire for healthy, undamaged hair in recent years, and there exist strong demands for hair-treatment compositions mainly consisting of non-synthetic ingredients that are able to repair damaged hairs as well as impart luster and excellent texture to hairs. For example, a hair-treatment composition comprising a saccharide, an oligopeptide, and an amino acid (See Patent Literature 4.) and a hair cosmetic composition comprising a disaccharide (For example, see Patent Literature 5.) have been proposed. Although these compositions showed the effect of reducing damages to hairs (hereinafter referred to as "hair damage") caused by treatments such as bleaching, perming and straightening or by high-temperature hair styling, it was uncertain whether the compositions could impart luster and excellent texture to hairs and they were not fully satisfactory in terms of their feeling of use and quality.

**Citation List**

**Patent Literature**

[Patent Literature 1] Japanese Patent Application Publication No. 2002-255756
[Patent Literature 2] Japanese Patent Application Publication No. H5-112437
[Patent Literature 3] Japanese Patent Application Publication No. 2006-232820
[Patent Literature 4] Japanese Patent Application Publication (Japanese Translation of PCT Application) No. 2009-519273
[Patent Literature 5] Japanese Patent Application Publication No. 2012-236804

**Disclosure of Invention**

**Technical Problem**

The present invention has been made to solve the above-mentioned drawbacks of conventional hair-setting agents or hair-setting compositions. An object of the present invention is to provide a novel hair-setting composition having sufficient hair-setting ability, and a means to obtain such a hair-setting composition. In another aspect, an object of the present invention is to provide a hair-setting composition which is excellent not only in a hair-setting ability but also in a feeling of use, flaking and/or treatment effect, and a means to obtain such a hair-setting composition.

**Solution to Problem**

The present inventors have accumulated intensive research efforts to attain the above object and unexpectedly found that an isomaltooligosaccharide, which is a type of saccharides, may be used as an active ingredient of an agent for enhancing a hair-setting ability which remarkably enhances the hair-setting ability and furthermore the hair-arranging ability of conventional hair-setting compositions. In addition, it was further found that a feeling of use and a flaking property of hair-setting compositions may be also remarkably improved and as far as a hair-treatment effect may be obtained by formulating an isomaltooligosaccharide in the hair-setting compositions. In other words, the present invention attains the above object by providing an agent for enhancing a hair-setting ability comprising an isomaltooligosaccharide as an active ingredient, and a hair-setting composition comprising the agent.

**Effects of Invention**

By formulating the agent for enhancing a hair-setting ability comprising an isomaltooligosaccharide as an active ingredient in accordance with one aspect of the present invention into a hair-setting composition, a hair-setting ability of the hair-setting composition can be remarkably enhanced. In another aspect, the agent for enhancing a hair-setting ability comprising an isomaltooligosaccharide as an active ingredient in accordance with one aspect of the present invention is advantageous not only in that it can enhance a hair-setting ability of a hair-setting composition which is formulated with the agent, but also in that it can improve a hair-arranging ability, feeling of use, and/or flaking property of the composition, and further in that it may be able to impart a hair-treatment ability to the composition.

**Brief Description of Drawings**

[Fig. 1] A figure showing the flaking state of the hair bundle to which Control Gel was applied.
[Fig. 2] A figure showing the flaking state of the hair bundle to which Test Gel comprising an isomaltooligosaccharide was applied.

**Description of Embodiments**

The agent for enhancing a hair-setting ability of the present invention is an agent for enhancing a hair-setting ability comprising an isomaltooligosaccharide as an active ingredient. The term "hair-setting" as used in the present description refers to setting and fixing hair, including holding a set hairstyle for a long period of time. As shown in the later-described experimental examples, the hair-setting ability may be evaluated, for example, by using the bending strength of a hair bundle applied with the agent for enhancing a hair-setting ability as an indicator. In other words, an enhancement of the hair-setting ability may be an increase of the bending strength of a hair bundle compared to when the agent for enhancing a hair-setting ability is not used.
In a preferred embodiment, the agent for enhancing a hair-setting ability comprising an isomaltooligosaccharide may also be able to enhance a hair-arranging ability, as shown in the later-described experimental examples. In other words, the agent for enhancing a hair-setting ability in accordance with a preferred embodiment of the present invention may be used as an agent for enhancing a hair-arranging ability. Herein, the term "hair-arranging" as used in the present description refers that a hairstyle can be easily changed even after the hairstyle is set. As shown in the later-described experimental examples, the hair-arranging ability may be evaluated, for example, by using the hair toughness as an indicator. In other words, an enhancement of the hair-arranging ability may be an increase of the hair toughness compared to when the agent for enhancing a hair-setting ability is not used.
In a preferred embodiment, the agent for enhancing a hair-setting ability of the present invention may not only enhance the hair-setting ability and/or the hair-arranging ability, but also impart an excellent feeling of use. "Excellent feeling of use" imparted by the agent for enhancing a hair-setting ability refers that a hair to which the hair-setting composition containing the agent for enhancing a hair-setting ability is applied has an excellent feel of hair. Herein, "feel of hair" may include stiffness of hair, squeakiness of hair, crunchy feel of hair, tangling of hair, stickiness of hair and/or smoothness of hair. In other words, "improving feel of hair" as used in the present description may include reducing stiffness, squeakiness, crunchy feel, tangling, and/or stickiness of hair and/or enhancing smoothness of hair. The improvement in feel of hair may be evaluated based on a sensory evaluation by a person skilled in the art, and may also be evaluated based on changes in the physical properties of hair, which is measured using an experimental equipment. For example, as shown in the later-described experimental examples, tangling of hair, stickiness of hair, and smoothness of hair may be evaluated using a frictional force of hair as an indicator. More specifically, tangling of hair, stickiness of hair, and smoothness of hair may be evaluated based on the maximum friction coefficient, mean dynamic friction coefficient, and the mean deviation of dynamic friction coefficient of hair when a probe (that mimics fingertips) moves on the surface of the hair.

In a preferred embodiment, the agent for enhancing a hair-setting ability of the present invention may be an agent for enhancing a hair-setting ability, which is characterized not only in that it enhances a hair-setting ability or hair-arranging ability of hair-setting compositions, but also in that it hardly causes flaking at the time of re-setting hair and it imparts an excellent appearance to the set hair. Herein, the term "flaking" refers to appearing of powder on the surface of a hair to which a hair-setting composition has been applied. Typically, it may be a state in which ingredients of the hair-setting composition get dried and solidified, thereby appearing on the surface of hair in a dusty manner. As shown in the later-described experimental examples, the agent for enhancing a hair-setting ability comprising an isomaltooligosaccharide as an active ingredient in accordance with a preferred embodiment of the present invention may remarkably inhibit the occurrence of flaking compared to when a hair-setting composition that does not contain an isomaltooligosaccharide is applied.

Meanwhile, as shown in the later-described experimental examples, an isomaltooligosaccharide may not only show an effect of enhancing a hair-setting ability and/or a hair-arranging ability but also show a hair-treatment effect. Therefore, in a preferred embodiment, the agent for enhancing a hair-setting ability of the present invention comprising an isomaltooligosaccharide may be preferably used as an agent for hair treatment. Herein, the agent for hair treatment comprising an isomaltooligosaccharide as an active ingredient in accordance with one embodiment of the present invention may be used for purpose of obtaining both an effect of enhancing a hair-setting ability and a hair-treatment effect. But it may be also used exclusively to obtain a hair-treatment effect.

Herein, the term "hair-treatment effect" refers to an effect of protecting hair from hair damage. The term "hair damage" refers to damages to hair caused by irradiation of ultraviolet rays contained in sunlight, etc., washing with shampoo, etc., drying with a hair dryer, perming, bleaching, dyeing, etc. Damages to hair may include, for example, a decrease of hair softness, a decrease of hair tensile strength, and a damage to the hair surface. Therefore, a protection of hair from hair damage may be a reduction or inhibition of a decrease of hair softness, a decrease of hair tensile strength, and the occurrence of a damage to the hair surface caused by ultraviolet rays, washing, drying, perming, bleaching, dyeing, etc., or may be an increase of decreased hair softness, hair tensile strength, and/or a reduction of a damage to the hair surface.

As shown in the later-described experimental examples, the softness of hair may be evaluated, for example, based on the bending strength of hair. Meanwhile, the tensile strength of hair may be evaluated based on the load applied to the hair when it is pulled in the longitudinal direction and breaks. The damage on the hair surface may be evaluated, for example, based on the adsorption area of a cationic fluorescence dye on the hair surface. Accordingly, in a preferred embodiment, the hair-treatment effect shown by the treatment agent in accordance with one embodiment of the present invention may be decreasing the bending strength of hair, increasing the tensile strength of hair, and/or decreasing the adsorption area of a cationic fluorescence dye on the hair surface, compared to when the treatment agent is not used.

For a user of the treatment agent, the hair-treatment effect, that is, the effect of protecting hair from hair damage, may be felt as the effect of maintaining a hair texture excellent or the effect of improving a hair texture. The hair-treatment effects that may be felt by a user of the treatment agent, that is, improvement of a hair texture, can be evaluated based on a sensory evaluation by a person skilled in the art, as shown in the later-described experimental examples. As indicators for evaluating a hair texture, for example, the suppleness, smoothness, silkiness, luster, and/or moistness may be used. Accordingly, the hair-treatment effect shown by the treatment agent comprising an isomaltooligosaccharide in accordance with one embodiment of the present invention may be an improvement of suppleness, smoothness, silkiness, luster, and/or moistness of hair compared to when the treatment agent is not used.

The term "isomaltooligosaccharide", which is used as an active ingredient of the agent for enhancing a hair-setting ability of the present invention, refers to a branched oligosaccharide containing glucose as a constituent sugar and having an $\alpha$-1,6 bond. Examples of such an isomaltooligosaccharide include but not limited to isomaltose, panose, isomaltotriose, isomaltotetraose, isomaltopentaose, and isomaltohexaose.

Typically, the isomaltooligosaccharide used in the present invention can be obtained by allowing a transferase to act on a starch syrup (mizuame) containing maltose as a main component. However, the isomaltooligosaccharide used in the present invention may be produced by any method. For example, the isomaltooligosaccharide produced by a fermentation method, an enzymatic method, an organic synthesis method, or the like may be used. Meanwhile, the isomaltooligosaccharide comprised in the agent for enhancing a hair-setting ability may be in any form. For example, the isomaltooligosaccharide in the form of liquid, semi-liquid, semi-solid, solid, powder, crystal, granule, etc. may be used.

Among the isomaltooligosaccharides that may be used in the present invention, panose is a water-soluble reducing trisaccharide and is also referred to as $\alpha$-D-glucopyranosyl-(1→6)-$\alpha$-D-glucopyranosyl-(1→4)-D-glucose. As methods for producing panose, methods for preparing a saccharide mixture containing panose, for example, by (1) a method in which $\alpha$-glucosidase (EC 3.2.1.20, also referred to as transglucosidase) is allowed to act on maltose, (2) a method in which dextransucrase (EC2.4.1.5) is allowed to act on a mixture of maltose and sucrose, and (3) a method in which an acid or an enzyme ($\alpha$-amylase, $\beta$-amylase, etc.) is allowed to act on pullulan or starch, are known. The obtained saccharide mixture may be further purified by an appropriate method, such as chromatography or crystallization, to prepare a high-panose-content product.

Among the isomaltooligosaccharides that may be used in the present invention, isomaltose, isomaltotriose, isomaltotetraose, isomaltopentaose, isomaltohexaose, and the like are water-soluble reducing oligosaccharides, which contain glucose as a constituent sugar and in which the constituent glucoses are linked by $\alpha$-1,6 bond. As a method for producing these isomaltooligosaccharides, for example, a method in which enzymes such as $\alpha$-amylase, $\beta$-amylase, and $\alpha$-glucosyltransferase are allowed to act on starch is known. In addition, isolation or purification by an appropriate method, such as chromatography and crystallization, may be further conducted.

The isomaltooligosaccharides comprised in the agent for enhancing a hair-setting ability of the present invention may not necessarily be highly purified as long as it can exhibit the desired effect, such as the effect of enhancing hair-setting ability. It may be in a partially purified form, in a form of an enzyme reaction solution, or in a form of an unseparated composition or a saccharide mixture containing other substances particular to the preparation method of isomaltooligosaccharides. The isomaltooligosaccharides comprised in the agent for enhancing a hair-setting ability of the present invention may be of any purity, but usually the one with a purity of 10% by mass or more, preferably with a purity of 20% by mass or more, more preferably with a purity of 40 to 99.99% by mass or more, in terms of a solid content, may be used. As a high-purity product, a high-purity isomaltooligosaccharide obtained through a crystallization process, or a high-purity isomaltooligosaccharide obtained through a chromatography process may be used. In a preferred embodiment, the isomaltooligosaccharide comprised in the agent for enhancing a hair-setting ability of the present invention may be a hydrogenated isomaltooligosaccharide.

The agent for enhancing a hair-setting ability of the present invention comprises an isomaltooligosaccharide, as described above, as an active ingredient. The agent for enhancing a hair-setting ability of the present invention may be in any form, including liquid form, semi-liquid form, semi-solid form, solid form, powder form, crystal form, granule form, tablet form, etc., for example. The action of enhancing a hair-setting ability, as used in the present invention, basically means the action of increasing a hair-setting ability, but it may be accompanied with one or more of the following actions: an action of imparting a smooth and non-sticky feeling of use to a hair-setting composition, an action of making it easy to set hair again, an action of reducing the occurrence of flaking when re-setting hair, an action of imparting an excellent appearance to hair, an action of softening hair after rinsing, an action of improving tensile strength, and an action of reducing damage on the hair surface.

The agent for enhancing a hair-setting ability of the present invention may comprise only one kind of isomaltooligosaccharide selected from isomaltose, panose, isomaltotriose, isomaltotetraose, isomaltopentaose, isomaltohexaose as an isomaltooligosaccharide. However, those comprise two or more kinds of isomaltooligosaccharides may be more preferable because the desired effect of the present invention, namely, the effect of enhancing a hair-setting ability, the effect of enhancing a hair-arranging ability, the effect of preventing flaking, the effect of improving a feeling of use and appearance, the effect of protecting hair after rinsing, and the hair-treatment effect, may be more remarkably shown. When the used isomaltooligosaccharides contain both isomaltose and panose, it may be particularly preferable because a particularly remarkable effect can be exhibited.

When the agent for enhancing a hair-setting ability of the present invention comprises both isomaltose and panose as isomaltooligosaccharides, their formulating ratio may be any, but as shown in the later-described experimental examples, the formulation ratio of isomaltose and panose may be preferably in the range of 5:1 to 1:15, more preferably in the range of 1:1 to 1:10 from the viewpoint of obtaining the hair-setting ability. From the viewpoint of obtaining a good feeling of use in addition to the hair-setting ability, the formulation ratio of isomaltose and panose may be preferably in the range of 1:3 to 1:10 and more preferably in the range of 1:3 to 1:5. Meanwhile, from the viewpoint of obtaining the hair-treatment effect, the formulation ratio of isomaltose and panose may be preferably in the range of 1:1 to 1:10 and more preferably in the range of 1:3 to 1:10.

The agent for enhancing a hair-setting ability of the present invention may contain any amount of an isomaltooligosaccharide as long as the desired effects of the present invention can be obtained. However, the total isomaltooligosaccharide content per solid in the agent may be usually 5% by mass or more and 100% by mass of less preferably 10% by mass or more and 100% by mass or less, more preferably 20% by mass or more and 100% by mass or less, and further preferably 30% by mass or more and 100% by mass or less. In a preferred embodiment, the upper limit of the total isomaltooligosaccharide content per solid in the agent for enhancing a hair-setting ability of the present invention may be usually 99% by mass or less, so that it can be provided industrially at relatively large scale, easily, and at low cost, 80% by mass or less to provide at lower cost, and 60% by mass or less to provide at even lower cost. The isomaltooligosaccharide that may be used in the present invention is as described above, but in a preferred embodiment, the isomaltooligosaccharide-containing saccharide (Product Name "Lissenare" (Registered Trademark)) (solid content of 74% by mass or more; isomaltooligosaccharide content per solid of 50% by mass or more; and panose content per solid of 28% by mass or more) commercialized by Hayashibara Co., Ltd. may be suitably used as the isomaltooligosaccharide to be comprised in the agent for enhancing hair-setting ability of the present invention, since it contains both isomaltose and panose in a preferable ratio.

In a preferred embodiment, the agent for enhancing a hair-setting ability of the present invention may be used by being formulated into or being comprised in a hair-setting composition. The term "hair-setting composition" as used in the present description refers to a hair-finishing cosmetics used for setting hair, fixing hair, and holding a hairstyle, which are used for purpose of improving luster, feel, texture, manageability, etc. of hair. Examples of a hair-setting composition include but not limited to hair-styling cosmetics, hair-care cosmetics, hair-washing cosmetics, permanent-wave cosmetics, hair-coloring cosmetics, hair-growth and nourishing cosmetics, etc. Examples of such hair finishing cosmetics include but not limited to hair-styling compositions such as hair water, hair mist, hair liquid, hair lotion, setting lotion, curler lotion, hair tonic, hair gel, hair foam, hair mousse, hair spray, hair serum, hair cream, hair wax, water grease, hair oil, pomade, tique, hair stick, and hair solid; hair-care compositions such as treatment lotion, hair serum, hair cream, hair blow, hair oil, coating for split ends, and antistatic composition; hair-washing compositions such as shampoo, dry shampoo, rinse, two-in-one shampoo, conditioner, hair treatment, and leave-in hair treatment; hair-permanent compositions such as permanent wave agent, straight perm agent, and hair straightening agent; hair-coloring compositions such as color stick, color spray, color rinse, color treatment, temporary hair dyes in liquid type, gel type, etc., hair dyes such as semi-permanent hair dye and permanent hair dye, and hair bleach; and compositions for hair growth such as hair nourishing agent and hair growth agent.

The hair-setting composition, to which the agent for enhancing a hair-setting ability of the present invention may be formulated into or comprised in, may be in any form and may be any of an aqueous solution system, a solubilized system, an emulsified system, a dispersion system, a solid system, or the like. Examples of forms of the hair-setting composition include but not limited to solid, liquid, mist, aerosol, gel, mousse, milky lotion, cream, and lotion. Whereas, a leave-on type form, which is not washed off, may be more preferable, it may be also in a rinse-off type form, which is washed off.

In the present description, making an isomaltooligosaccharide be comprised in an agent for enhancing a hair-setting ability or making the agent for enhancing a hair-setting ability comprising an isomaltooligosaccharide be formulated in a hair-setting composition means making the isomaltooligosaccharide or the agent for enhancing a hair-setting ability comprising an isomaltooligosaccharide be comprised in or be blended in an existing product or at an appropriate step during production process of from raw materials to products of the agent for enhancing a hair-setting ability or the hair-setting composition, by appropriately combining one or more methods including mixing, kneading, dissolving, melting, dispersing, suspending, emulsification, immersion, permeation, spreading, applying, coating, spraying, injection, crystallization, solidification, reverse micellization, etc. The step of making the isomaltooligosaccharide or the agent for enhancing a hair-setting ability be comprised in or be blended in by these methods may be performed under normal pressure, high pressure, and reduced pressure, and further under supercritical condition using supercritical fluid.

The content of the isomaltooligosaccharide in the hair-setting composition comprising the agent for enhancing a hair-setting ability of the present invention may be any as long as the isomaltooligosaccharide comprised therein can exert the desired effect, such as the effect of enhancing a hair-setting ability. However, the isomaltooligosaccharide content in the hair-setting composition may be usually in the range of 0.1 to 30.0% by mass, preferably in the range of 0.1 to 25.0% by mass, more preferably in the range of 0.5 to 20.0% by mass, and further preferably in the range of 1 to 10.0% by mass, based on the total solid content of the isomaltooligosaccharide. If the isomaltooligosaccharide content in the hair-setting composition is less than 0.1% by mass, it may not be preferable since the effect of enhancing a hair-setting ability shown by the isomaltooligosaccharide may not be sufficient. On the other hand, if the isomaltooligosaccharide content in the hair-setting composition is more than 30.0% by mass, it may not be preferable since the feeling of use may become worse.

In an embodiment, when the hair-setting composition comprising the agent for enhancing a hair-setting ability of the present invention contains a substance having an amino group in its molecular structure, such as an amino acid, the quality of the hair-setting composition may be expected to be deteriorated, for example, due to the Maillard reaction between the reducing end of an isomaltooligosaccharide and amino group. In such a case, a hydrogenated isomaltooligosaccharide with decreased reducing ability may be advantageously used as an isomaltooligosaccharide to be comprised in the agent for enhancing a hair-setting ability.

In a preferred embodiment, the agent for enhancing a hair-setting ability of the present invention may be used in place of a part or the whole of an ingredient having a hair-setting ability, such as water-soluble polymers used in hair-setting compositions in general.

When the agent for enhancing the hair-setting ability of the present invention is comprised in hair-setting compositions, not only the hair-setting ability of the hair-setting composition may be enhanced but also the hair-arranging ability may be enhanced, and furthermore discomfort during use or after being washed off may be improved. In a preferred embodiment, hair-setting compositions comprising the agent for enhancing a hair-setting ability of the present invention leaves hair not sticky after use and gives elasticity to hair, making it easier to manage, particularly when they are used in soft hair, compared to hair-setting compositions not containing the agent for enhancing a hair-setting ability of the present invention. In a further preferred embodiment, hair-setting compositions comprising the agent for enhancing the hair-setting ability of the present invention show the excellent hair-treatment effect and/or hair-protecting effect that hair is softened, the tensile strength of hair is increased, and a damage on the hair surface is reduced after being washed off. Furthermore, an isomaltooligosaccharide comprised in the agent for enhancing a hair-setting ability of the present invention has an effect of moisturizing the skin and the effect of preventing denaturation of proteins. Accordingly, by formulating the agent for enhancing a hair-setting ability of the present invention, the irritation and the occurrence of itching and rashes on the scalp and the skin caused by irritating factors etc., such as surfactants, which may be contained in minute amounts in hair-setting compositions, may be advantageously suppressed or prevented.

In addition to an isomaltooligosaccharide, the hair-setting composition in accordance with the present invention may further contain one or more of the ingredients generally used in hair-setting compositions, including ingredients for imparting a hair-setting ability, depending on the form and purpose of the hair-setting composition, within a quantitative and qualitative range that does not impair the effects of the present invention. Examples of such optional ingredients include but not limited to water-soluble polymers, water-soluble polyhydric alcohols, liquid paraffin, squalane, lanolin derivatives, higher alcohols, various ester oils, avocado oils, palm oils, silicone oils, high-molecular-weight silicone oils, polyalkylene glycol polyethers and their carboxylic acid oligoester compounds, oils such as terpene hydrocarbons, moisturizers such as pyrrolidone carboxylic acid salts, UV absorbents, resins such as acrylic resins, silicone resins, and polyvinylpyrrolidone, proteins or protein hydrolysates such as soybean protein, gelatin, collagen, silk fibroin, and elastin, preservatives such as ethylparaben and butylparaben, nutrient agents such as amino acids, biotin, and pantothenic acid derivatives, antiseborrheic agents such as γ-oryzanol, sodium dextran sulfate, and vitamin E, diluents such as water, ethanol, isopropanol, and tetrachlorodifluoroethane and other ingredients such as substances that are used as raw materials of cosmetics or quasi-drugs, pharmaceuticals, antibacterial agents, antioxidants, fragrances, coloring agents, preservatives, chelating agents, cooling agents, vitamins, plant extracts, aerosol propellants, pH adjusting agents, and surfactants. Furthermore, other substances that are used as raw materials of external dermal agents, for example the ones described in Patent Literature 3 may be also included.

The water-soluble polymers that may be appropriately comprised include but not limited to natural polymers, semi-synthetic polymers, synthetic polymers, and the like. Examples of natural polymers include but not limited to gum arabic, tragacanth gum, guar gum, locust bean gum, karaya gum, Irish moss, quince seed, pullulan, gelatin, shellac, rosin, casein, mucopolysaccharides such as hyaluronic acid and chondroitin sulfate, and their salts, etc. Examples of semi-synthetic polymers include but not limited to carboxymethylcellulose sodium salt, hydroxyethylcellulose, methylcellulose, ethylcellulose, hydroxypropylcellulose, crystalline cellulose, etc. Examples of synthetic polymers include but not limited to polyvinyl alcohol, (vinylpyrrolidone/VA) copolymer, sodium polyacrylate, carboxyvinyl polymer (carbomer), polyvinylmethylcellulose, polyamide resin, silicone, etc. These water-soluble polymers may be used alone or in combination of two or more if appropriate. If a water-soluble polymer is comprised in the hair-setting composition, the water-soluble polymer content in the hair-setting composition may be any but may be in the range of 0.001% by mass to 10.0% by mass or preferably in a range of 0.01% by mass to 7.0% by mass, based on the total mass of the hair-setting composition.

Examples of water-soluble polyhydric alcohols that may be appropriately comprised include but not limited to ethylene glycol, diethylene glycol, triethylene glycol, polyethylene glycol, propylene glycol, dipropylene glycol, tripropylene glycol, 1,3-butylene glycol, polybutylene glycol, glycerin, diglycerin, polyglycerin, sorbitol, maltitol, mannitol and derivatives thereof. In a preferred embodiment, 1,3-butylene glycol, sorbitol, mannitol, propylene glycol, dipropylene glycol, glycerin, and derivatives of glycerin may be preferably used. These water-soluble polyhydric alcohols may be used alone or in combination of two or more, if appropriate. The water-soluble polyhydric alcohol content in the hair setting composition may be any but may be in the range of 0.01% by mass to 20.0% by mass or preferably in a range of 1.0% by mass to 10.0% by mass, based on the total mass of the hair setting composition.

In a preferred embodiment, the hair-setting composition of the present invention may be mixed with a solvent such as water and an alcohol to obtain an aerosol stock solution and may be used as an aerosol-type hair-setting composition using the aerosol stock solution together with an aerosol propellant.

As an aerosol propellant, any aerosol propellant that may be used for aerosol-type cosmetics may be used. Examples of aerosol propellants include but not limited to dichlorodifluoromethane, difluoromonochloroethane, monochlorodifluoromethane, dichlorotetrafluoroethane, tetrafluoroethane, dimethyl ether (DME), propane, isobutane, n-butane, isopentane, liquefied petroleum gas (LPG; containing propane, isobutane, n-butane as main components), nitrogen gas, carbon dioxide gas, or a mixture thereof. These aerosol propellants may be used alone or in combination of two or more if appropriate.

The aerosol-type hair-setting composition of the present invention may be produced in any method. For example, the aerosol-type hair-setting composition of the present invention may be prepared by dissolving an isomaltooligosaccharide in a solvent, optionally blending other additives, such as a surfactant, therein, followed by emulsification (dispersing), and then adding an aerosol propellant therein. Herein, the isomaltooligosaccharide-containing composition and the propellant are usually filled into a conventional pressure container for aerosol in accordance with a conventional method.

Below, the present invention is described in more detail with reference to experiments.

<Experiment 1: Test for Hair Setting Ability>

In order to examine the influence of an isomaltooligosaccharide on a hair-setting ability, hair-setting compositions in liquid form comprising an isomaltooligosaccharide were prepared and their hair-setting abilities were evaluated. As an isomaltooligosaccharide, the isomaltooligosaccharide-containing saccharide solution prepared in below described Experiment 1-1, isomaltose (Syrup Form; Solid Content 76.4% by mass; Isomaltose Purity 98.2% by mass; Hayashibara Co., Ltd., Okayama, Japan) and panose (Powder Form; Panose Purity 97.6% by mass; Hayashibara Co., Ltd., Okayama, Japan) were used.

<Experiment 1-1: Preparation of Isomaltooligosaccharide-containing Saccharide Solution>

In accordance with the method described in Experiment 1 of Japanese Patent Application Publication No. H4-360663, an isomaltooligosaccharide-containing saccharide solution was prepared. Briefly, maltose in powder form (Product Name "SUNMALT" (Registered Trademark), Hayashibara Co., Ltd., Okayama, Japan) was prepared into an aqueous solution with a concentration of 30% by mass. To this aqueous solution, an enzyme preparation (Product Name "Transglucosidase L "Amano"", Amano Enzyme Inc., Aichi, Japan) containing $\alpha$-glucosidase was added in an amount of 0.08% by mass of solid isomaltose, and the pH of the solution was adjusted to 5.5. The solution was allowed to react at 55°C for 18 hours, followed by heat-inactivation of the enzyme. In accordance with the conventional method, the obtained solution was purified through filtration, decoloration, and desalting using ion-exchange resins (H type and OH type), and then concentrated to obtain an isomaltooligosaccharide-containing saccharide solution with a solid content of 80% by mass. As a result of analysis in accordance with the conventional method, the saccharide composition per solid of this isomaltooligosaccharide-containing saccharide solution was determined to be 21.4% by mass of glucose, 17.6% by mass of maltose, 9.9% by mass of isomaltose, 4.3% by mass of maltotriose, 29.8% by mass of panose, 1.7% by mass of isomaltotoriose, and 15.3% by mass of high-molecular weight saccharides including tetrasaccharide or higher isomaltooligosaccharides. Herein, the isomaltooligosaccharide content per solid was 41.4% by mass or more.

<Experiment 1-2: Preparation of Test Compositions>

To the isomaltooligosaccharide-containing saccharide solution (100 g of isomaltooligosaccharide in terms of solid) prepared in Experiment 1-1, ultrapure water was added so that the total amount became 700 g. The mixture was uniformly mixed using a stirrer to obtain an isomaltooligosaccharide solution. Next, to 100 g of ethanol, 50 g of (vinylpyrrolidone/VA) copolymer (Product Name "Luviskol VA64P", BASF Japan Ltd.) was added as a resin having a hair-setting ability, and then 4 g of phenoxyethanol was added as a preservative. The mixture was stirred using a stirrer so the added compounds were dissolved to obtain a (vinylpyrrolidone/VA) copolymer solution. The (vinylpyrrolidone/VA) copolymer solution was added to the isomaltooligosaccharide solution, and the mixture was uniformly mixed using a stirrer. Ultrapure water was added to the mixture so that the total amount became 1,000 g to obtain Test Composition 1 in liquid form containing an isomaltooligosaccharide at a final concentration of 10% by mass in terms of a solid content. Meanwhile, Test Composition 2 was prepared in the same procedure as in Test Composition 1 except that ultrapure water was added to 131 g of the above-described isomaltose in syrup form (100 g in terms of solid) instead of the isomaltooligosaccharide-containing saccharide solution prepared in Experiment 1-1. Test Composition 3 was prepared in the same procedure as in Test Composition 1 except that ultrapure water was added to 100 g of the above-described panose in powder form instead of the isomaltooligosaccharide-containing saccharide solution prepared in Experiment 1-1. Test Composition 4 was prepared in the same procedure as in Test Composition 1 except that ultrapure water was added to 32.8 g of the above-described isomaltose in syrup form (25 g in terms of solid amount) and 75 g of the above-described panose in powder form instead of the isomaltooligosaccharide-containing saccharide solution prepared in Experiment 1-1. On the other hand, Control Composition 1 in a liquid composition form was prepared in the same procedure in Test Composition 1 except that ultrapure water was used instead of the isomaltooligosaccharide-containing saccharide solution. The compositions of Test Compositions 1 to 4 and Control Composition 1 were shown in Table 1.

[Table 1]

| | Control Composition 1 | Test Composition 1 | Test Composition 2 | Test Composition 3 | Test Composition 4 |
|---|---|---|---|---|---|
| Isomaltooligosaccharide-containing saccharide solution prepared in Experiment 1-1 (in terms of solid) | - | 100 g | - | - | - |
| Isomaltose (in terms of solid) | - | - | 100 g | - | 25 g |
| Panose | - | - | - | 100 g | 75 g |
| Ethanol | 100 g | 100 g | 100 g | 100 g | 100 g |
| (vinylpyrrolidone/VA) copolymer | 50 g | 50 g | 50 g | 50 g | 50 g |
| Phenoxyethanol | 4g | 4g | 4g | 4g | 4g |
| Ultrapure water | Bal. | Bal. | Bal. | Bal. | Bal. |
| Total | 1,000 g | 1,000 g | 1,000 g | 1,000 g | 1,000 g |

<Experiment 1-3: Preparation of Hair Bundles for Measuring Hair-Setting Ability>

Hair strand (Product Name "Human black hair BS-B3N"; Beaulax Co., Ltd., Saitama, Japan) was immersed in an aqueous solution containing 9% by mass of sodium laureth sulfate (prepared by diluting sodium laureth sulfate (Product Name "EMAL E-27C", Kao Corporation, Tokyo Japan) 3-fold with ultrapure water) for 1 hour. After immersion, the hair strand was rinsed thoroughly under running water using tap water and subsequently ultrapure water. The hair strand was completely dried using a cold air dryer and then naturally dried overnight at room temperature. The dried hair strand was divided into 1 g portions, which were bundled with a rubber band to obtain hair bundles. The ends of the divided hair bundles were trimmed at 15 cm below the rubber band, and the trimmed hair bundles were used for the test. On 1 g of the hair bundle, 0.5 g of Test Composition 1, 2, 3, or 4 was dropped and then evenly applied by hand. After application of test compositions, the hair bundles were wrapped in aluminum foil so as to have a width of 1 cm, formed into a flat plate shape by applying a constant pressure from above, and incubated in a constant temperature and humidity chamber (22°C, relative humidity 50%) for 1 hour. After 1 hour, the hair bundle was taken out from aluminum foil, dried for 24 hours while hanging on a rack in the above-described constant temperature and humidity chamber, and used for measuring a hair-setting ability as a hair bundle applied with Test Composition 1, 2, 3 or 4. Meanwhile, a hair bundle applied with Control Composition 1 was prepared in the same procedure as described above except that Control Composition 1 was applied instead of Test Composition 1, 2, 3 or 4.

<Experiment 1-4: Measurement of Hair-Setting Ability>

The hair-setting ability was evaluated using the bending strength of hair bundles applied with Test Compositions or Control Composition 1 (hereinafter may be also referred to as "Test Composition-applied hair bundle" or "Control Composition 1-applied hair bundle") as an indicator. The bending strengths of the hair bundles were measured using a rheometer (Product Name "CR-500DX-SII", Sun Scientific Co., Ltd., Tokyo, Japan). Briefly, the Test Composition-applied hair bundles and Control Composition-applied hair bundles were extended over two measurement tables with a width of 1 cm and a height of 5 cm and having horizontal top surfaces, which were set at an interval of 7.8 cm. The pressure was applied to the extended hair bundles using a metal probe with a wedge-shaped jig (Product Name: "Pressure Sensitive Axis No. 34 Tooth Type (A)", Sun Scientific Co., Ltd., Tokyo Japan), while lowering the probe vertically at a speed of 60 mm/min. After the probe contacted the hair bundle, the probe was further lowered for 3 cm. The load was measured every 50 msec. It was deemed that the hair bundle was broken when the maximum load was shown after the probe contacted the hair bundle, and the bending strength of the hair bundle was defined as the load (N) when the maximum load was shown after the probe contacted the hair bundle. Measurements of the load were performed three times for each of Test Compositions and Control Composition using three similarly prepared hair bundles. Table 2 shows mean and standard deviation of the maximum load when the hair bundle was broken (the bending strength) which was measured for the hair bundles applied with Test Compositions or Control Composition 1.

<Statistical Analysis>

Measured values were expressed as mean $\pm$ standard deviation (SD). Statistical analysis was performed by unpaired t-test. A significance level (p value) of less than 0.05 (less than 5% risk) was judged to be significant.

[Table 2]

|  | Bending Strength (N) |
|---|---|
| Hair Bundle Applied with Control Composition 1 | $0.28 \pm 0.04$ |
| Hair Bundle Applied with Test Composition 1 | $0.59 \pm 0.16$' |
| Hair Bundle Applied with Test Composition 2 | $0.43 \pm 0.09$' |
| Hair Bundle Applied with Test Composition 3 | $0.51 \pm 0.04$** |
| Hair Bundle Applied with Test Composition 4 | $0.73 \pm 0.15$** |
| * : P<0.05 vs Hair Bundle Applied with Control Composition 1 <br> ** :P<0.01 vs Hair Bundle Applied with Control Composition 1 | |

The hair bundles applied with either one of Test Compositions 1 to 4 comprising the same amount of isomaltooligosaccharide in terms of solid content were prepared and their hair-setting abilities were evaluated. As shown in Table 2, for the hair bundle applied with Control Composition 1, which was prepared in the same manner as Test Compositions except that it does not contain isomaltooligosaccharides, the maximum load when the hair bundle was broken (hereinafter may be referred to simply as "the bending strength") was $0.28\pm0.04$ N. Meanwhile, the bending strengths of the hair bundles applied with Test Compositions 1, 2, 3 or 4, containing the isomaltooligosaccharide-containing saccharide solution prepared in Experiment 1-1, isomaltose, panose, or both isomaltose and panose, respectively, were $0.59\pm0.16$ N, $0.43\pm0.09$ N, $0.51\pm0.04$ N, and $0.73\pm0.15$ N, respectively, revealing that the bending strengths of the hair bundles applied with Test Compositions 1 to 4 were remarkably increased compared to that of the hair bundles applied with Control Composition 1. Furthermore, it was revealed that the bending strength of the hair bundle applied with Test Composition 1 comprising the isomaltooligosaccharide-containing saccharide solution prepared in Experiment 1-1 was further remarkably increased even compared to that of the hair bundle applied with Test Composition 2 comprising only isomaltose as an isomaltooligosaccharide and that of the hair bundle applied with Test Composition 3 comprising only panose as an isomaltooligosaccharide. Moreover, it was revealed that the bending strength was further remarkably increased in the hair bundle applied with Test Composition 4, which was prepared by blending both isomaltose and panose so that the composition contained isomaltose and panose at essentially the same mass ratio of isomaltose to panose (1:3) as the mass ratio of isomaltose to panose (9.9% by mass of isomaltose:29.8% by mass of panose) in the isomaltooligosaccharide-containing saccharide solution prepared in Experiment 1-1. This result indicates that the hair bundle is remarkably hardened, in other words, the hair-setting ability is remarkably enhanced when being applied with Test Composition 1 or Test Composition 4 comprising both isomaltose and panose, compared to when only isomaltose or only panose is used as an isomaltooligosaccharide. Meanwhile, the bending strength was higher in the hair bundle applied with Test Composition 4, which was prepared by formulating only isomaltose (Purity 98.2%) and panose (Purity: 97.6%) as isomaltooligosaccharides at a mass ratio of isomaltose to panose of 1:3, than the hair bundle applied with Test Composition 1, prepared by formulating, as an isomaltooligosaccharide, the isomaltooligosaccharide-containing saccharide solution (Isomaltooligosaccharide content of 41.4% by mass or more) prepared in Experiment 1-1, which contains saccharide other than isomaltooligosaccharides. This result suggests that isomaltooligosaccharides are the ingredient having an effect of remarkably hardening hair bundles, in other words, an active ingredient of enhancing the hair-setting ability. The reasons why isomaltooligosaccharides are more effective in hardening hair bundles than other saccharide remain to be elucidated but may be because the branch structure peculiar to isomaltooligosaccharides penetrates into gaps of hair cuticles and increases the strength of hair. In addition, it is presumed that the isomaltooligosaccharides coated on hair bundles increase the adhesion between hairs through electrostatic interactions, such as hydrogen bonding via hydroxyl groups, and contribute to the enhancement of a hair-setting ability. Because isomaltooligosaccharides have the branch structure via $\alpha$-1,6 bond, which is not present in saccharides other than isomaltooligosaccharides (glucose, maltose, maltotriose, etc.) contained in the isomaltooligosaccharide-containing saccharide solution prepared in Experiment 1-1, it is thought that this branch structure plays an important role in enhancing the hair-setting ability.

<Experiment 2: Effect of Formulating Ratio of Isomaltose and Panose on Hair-Setting Ability>

Hair-Setting Compositions comprising as isomaltooligosaccahrides isomaltose and panose at different formulating ratios were prepared and their hair-setting abilities were evaluated.

<Experiment 2-1: Preparation of Test Compositions>

Test Compositions 5 to 13 in liquid form were prepared in the same procedure as in Experiment 1-2 except that 9 kinds of isomaltooligosaccharide mixtures with different mixing ratios of isomaltose and panose, which were obtained by mixing isomaltose and panose at the mass ratio shown in Table 3, were used as isomaltooligosaccharide (in other words, the mass ratios of isomaltose to panose in Test Compositions 5 to 13 described later were adjusted to 100:0, 83:17, 75:25, 50:50, 25:75, 17:83, 9:91, 6:94, and 0: 100, respectively) and that the final concentration of the isomaltooligosaccharide mixture in the test compositions was set to 3% by mass. On the other hand, Control Composition 2 in a liquid composition form was prepared in the same procedure as described for Test Compositions 5 to 13 except that ultrapure water was used instead of the isomaltooligosaccharide mixture. The compositions of Test Compositions 5 to 13 and Control Composition 2 were shown in Table 3.

[Table 3]

| | Control Composition 2 | Test Composition | | | | | | | | |
|---|---|---|---|---|---|---|---|---|---|---|
| | | 5 | 6 | 7 | 8 | 9 | 10 | 11 | 12 | 13 |
| Isomaltose (in terms of solid) | - | 30 g | 25 g | 22.5 g | 15g | 7.5 g | 5g | 2.7 g | 1.9 g | - |
| Panose | - | - | 5g | 7.5 g | 15g | 22.5 g | 25 g | 27.3 g | 28.1 g | 30 g |
| Ethanol | 100 g | 100 g | 100 g | 100 g | 100 g | 100 g | 100 g | 100 g | 100 g | 100 g |
| (vinylpyrrolidone/ VA) copolymer | 50 g | 50 g | 50 g | 50 g | 50 g | 50 g | 50 g | 50 g | 50 g | 50 g |
| Phenoxyethanol | 4g | 4g | 4g | 4g | 4g | 4g | 4g | 4g | 4g | 4g |
| Ultrapure water | Bal. | Bal. | Bal. | Bal. | Bal. | Bal. | Bal. | Bal. | Bal. | Bal. |
| Total | 1,000 g | 1,000 g | 1,000 g | 1,000 g | 1,000 g | 1,000 g | 1,000 g | 1,000 g | 1,000 g | 1,000 g |

<Experiment 2-2: Preparation of Test Hair Bundles and Measurement of Hair-Setting Ability>

Test hair bundles were prepared in the same manner as in Experiment 1-3 except that Test Compositions 5 to 13 in liquid form containing the isomaltooligosaccharide mixture or Control Composition 2 in liquid form not containing the isomaltooligosaccharide mixture were used instead of Test Compositions 1 to 4 in liquid form containing the isomaltooligosaccharide or Control Composition 1 in liquid form not containing the isomaltooligosaccharide. The measurements of the hair-setting ability for the test hair bundles were carried out following the procedure described in Experiment 1-3. Table 4 shows the maximum load when the hair bundles were broken (the bending strength) which were measured for the hair bundles applied with Test Compositions or Control Composition 2.

[Table 4]

| | Mass ratio of isomaltose : panose in the applied compositions (in terms of solid) | Bending Strength (N) |
|---|---|---|
| Hair Bundle Applied with Control Composition 2 | - | 0.13 |
| Hair Bundle Applied with Test Composition 5 | 100:0 | 0.14 |
| Hair Bundle Applied with Test Composition 6 | 83:17 | 0.29 |
| Hair Bundle Applied with Test Composition 7 | 75:25 | 0.31 |
| Hair Bundle Applied with Test Composition 8 | 50:50 | 0.78 |
| Hair Bundle Applied with Test Composition 9 | 25:75 | 0.55 |
| Hair Bundle Applied with Test Composition 10 | 17:83 | 0.55 |
| Hair Bundle Applied with Test Composition 11 | 9:91 | 0.52 |
| Hair Bundle Applied with Test Composition 12 | 6:94 | 0.34 |
| Hair Bundle Applied with Test Composition 13 | 0:100 | 0.23 |

The hair bundles applied with either one of Test Compositions 5 to 13 in liquid form, which contained the same amount of isomaltooligosaccharide in terms of solid but contained isomaltose and panose as isomaltooligosaccharide at different mass ratios of 100:0 (1:0), 83:17 (5:1), 75:25 (3:1), 50:50 (1:1), 25:75 (1:3), 17:83 (1:5), 9:91 (1:10), 6:94 (1:15), and 0:100 (0:1), were prepared and their hair-setting ability was evaluated. As shown in Table 4, the bending strength was 0.13 N for the hair bundle applied with Control Composition 2, which was prepared in the same manner as Test Compositions 5 to 13 except that it does not contain isomaltooligosaccharides. Meanwhile, the bending strengths of the hair bundles applied with Test Compositions 5 to 13 containing isomaltose and/or panose as isomaltooligosaccharide were 0.14 N, 0.29 N, 0,31 N, 0.78 N, 0.55 N, 0.55 N, 0.52 N, 0.34N and 0.23 N, respectively, revealing that the bending strengths of the hair bundles were increased by applying any of Test Compositions 5 to 13 comprising either isomaltose, isomaltose and panose, or panose as isomaltooligosaccharide. Furthermore, it was revealed that the bending strength of the hair bundles applied with Test Compositions 6 to 12 containing both isomaltose and panose (in other words, Test Compositions containing isomaltose and panose at a mass ratio of isomaltose to panose in the range of 5:1 to 1:15 in terms of solid content) was remarkably increased compared to that of the hair bundle applied with Test Composition 5 containing only isomaltose as an isomaltooligosaccharide, or that of the hair bundle applied with Test Composition 13 containing only panose as an isomaltooligosaccharide. This result indicates that the hair bundle is remarkably hardened, in other words, the hair-setting ability is remarkably enhanced when the mass ratio of isomaltose to panose is in the range of 5:1 to 1:15 in terms of the solid content.

Moreover, it was revealed that the bending strength of the hair bundles applied with Test Compositions 8 to 11 containing both isomaltose and panose (in other words, Test Compositions containing isomaltose and panose at a mass ratio of isomaltose to panose in the range of 1:1 to 1:10 in terms of solid content) was further remarkably increased. This result indicates that the hair bundle is further remarkably hardened, in other words, the hair-setting ability is further remarkably enhanced when the mass ratio of isomaltose to panose is in the range of 1:1 to 1:10 in terms of the solid content.

<Experiment 2-3: Effect of Formulating Ratio of Isomaltose and Panose on Feeling of Use of Test Composition in Liquid Form Containing Isomaltooligosaccharide Mixture and on Property of Test Hair Bundles Applied with Test Composition>

Using Test Compositions 5 to 13 and Control Composition 2 obtained in Experiment 2-1, the feeling of use when the compositions were applied to hair bundles and the properties of hair bundles applied with the compositions were evaluated. The test hair bundles were prepared in the same manner as in Experiment 2-2. The test hair bundles were combed 5 time using a horizontal comb made of polypropylene (comb tooth spacing: 2mm). Thereafter, the feeling of use (film-forming texture, stickiness) and the property (flaking after combing) when the composition was applied to the hair bundles were evaluated by 6 trained panelists by visually inspecting the test hair bundles as well as touching it with bare hands.

The evaluation was made on the following three items: "film-forming texture", "stickiness", and "flaking after combing". "Film-forming texture" refers to the stiff, squeak, or crunchy feeling due to the excessive hardness of hair bundle caused by application of Test Compositions, while having the necessary hair-setting ability to hold a hair styling, as compared to hair to which Test Composition was not applied. This was evaluated by the degree of discomfort. "Stickiness" was evaluated by the degree of discomfort of unpleasant stickiness caused by application of Test Compositions. As to "flaking after combing", the amount of resin wastes contained in Test Compositions that were peeled off by combing after application of Test Compositions was visually evaluated.

Evaluation for each item was carried out on a 5-point scale based on the criteria of "5: very good", "4: good", "3: neither good nor bad", "2: bad", and "1: very bad". The average point was calculated by dividing the sum of the points of all panelists by the number of panelists. It was judged ◎ when the average point was 4 points or more, O when the average point was 3 points or more, △ when the average point was 2 points or more, and × when the average point was less than 2 points. The obtained results were shown in Table 5.

[Table 5]

| | Mass ratio of isomaltose : panose in the applied composition (in terms of solid content) | Film-Forming Texture | Stickiness | Flaking after Combing | Overall Evaluation |
|---|---|---|---|---|---|
| Hair Bundle Applied with Control Composition 2 | - | ○ | ◎ | ○ | ○ |
| Hair Bundle Applied with Test Composition 5 | 100:0 | × | × | △ | × |
| Hair Bundle Applied with Test Composition 6 | 83:17 | × | × | × | × |
| Hair Bundle Applied with Test Composition 7 | 75:25 | × | × | △ | × |
| Hair Bundle Applied with Test Composition 8 | 50:50 | ○ | ○ | △ | △ |
| Hair Bundle Applied with Test Composition 9 | 25:75 | ◎ | ◎ | ◎ | ◎ |
| Hair Bundle Applied with Test Composition 10 | 17:83 | ◎ | ○ | ◎ | ◎ |

(continued)

|  | Mass ratio of isomaltose : panose in the applied composition (in terms of solid content) | Film-Forming Texture | Stickiness | Flaking after Combing | Overall Evaluation |
|---|---|---|---|---|---|
| Hair Bundle Applied with Test Composition 11 | 9:91 | ○ | ○ | ○ | ○ |
| Hair Bundle Applied with Test Composition 12 | 6:94 | △ | △ | △ | △ |
| Hair Bundle Applied with Test Composition 13 | 0:100 | × | △ | × | × |

As shown in Table 5, as a result of the evaluation using isomaltose and/or panose as isomaltooligosaccharides and with the hair bundles applied with the test compositions in liquid form prepared by varying the mass ratio of isomaltose and panose in the range of 100:0 to 0:100, the feeling of use (film-forming texture) was judged favorable (i.e., judged "○" or "◎") when the mass ratio of isomaltose and panose was in the range of 1:1 to 1:10, and was judged very favorable (i.e., judged "◎") when the mass ratio of isomaltose and panose was in the range of 1:3 to 1:5. Meanwhile, the feeling of use (stickiness) was judged favorable (i.e., judged "○" or "◎") when the mass ratio of isomaltose and panose was in the range of 1:1 to 1:10, and was judged very favorable (i.e., judged OO ") when the mass ratio of isomaltose and panose was 1:3. The property (flaking after combing) was judged favorable (i.e., judged "○" or "◎") when the mass ratio of isomaltose and panose was in the range of 1:3 to 1:10, and was judged very favorable (i.e., judged "◎") when the mass ratio of isomaltose and panose was in the range of 1:3 to 1:5. Overall evaluation was judged favorable (i.e., judged "○" or "◎") when the mass ratio of isomaltose and panose was in the range of 1:3 to 1:10, and was judged very favorable (i.e., judged "◎") when the mass ratio of isomaltose and panose was in the range of 1:3 to 1:5. This result indicates that when the two kinds of isomaltooligosaccharides, namely isomaltose and panose, are used as active ingredients of the agent for enhancing a hair-setting ability, the good feeling of use and property are obtained at the mass ratio of isomaltose and panose in the range of 1:3 to 1:10, and that these effects are particularly prominent when the mass ratio of isomaltose and panose is in the range of 1:3 to 1:5.

<Experiment 3: Effect of Isomaltooligosaccharide Content on Feeling of Use of Hair-Setting Compositions and on Property of Hair Bundles Applied with Hair-Setting Compositions>

While keeping the formulating ratio (mass ratio) of isomaltose and panose as isomaltooligosaccharides fixed, hair-setting compositions which defer from one another only in the varied isomaltooligosaccharide contents were prepared, and their feeling of use and the property of hair bundles when the hair-setting compositions were applied to the hair bundles were evaluated.

<Experiment 3-1: Preparation of Test Compositions>

Test Compositions 14 to 21 were prepared in the same manner as in Experiment 1-2 except that an isomaltooligosaccharide mixture containing isomaltose and panose at the mass ratio of 1:3 was used as an isomaltooligosaccharide and that the final concentration of isomaltooligosaccharide in the test compositions was set to be in the range of 0.1 to 40% by mass (in other words, the final concentration of the isomaltooligosaccharide in the later-described Test Compositions 14 to 21 were set to be 0.1, 0.5, 1, 5, 10, 20, 25, and 30% by mass, respectively). On the other hand, Control Composition 3 in a liquid composition form was prepared in the same manner as Test Compositions 14 to 21 except that ultrapure water was used instead of the isomaltooligosaccharide mixture. The compositions of Test Compositions 14 to 21 and Control Composition 3 were shown in Table 6.

[Table 6]

| | Control Composition 3 | Test Composition | | | | | | | |
|---|---|---|---|---|---|---|---|---|---|
| | | 14 | 15 | 16 | 17 | 18 | 19 | 20 | 21 |
| Isomaltose (in terms of solid) | - | 0.25 g | 1.25 g | 2.5 g | 12.5 g | 25 g | 50 g | 62.5 g | 75 g |
| Panose | - | 0.75 g | 3.75 g | 7.5 g | 37.5 g | 75 g | 150 g | 187.5 g | 225 g |
| Ethanol | 100 g | 100 g | 100 g | 100 g | 100 g | 100 g | 100 g | 100 g | 100 g |
| (vinylpyrrolidone/ VA) copolymer | 50 g | 50 g | 50 g | 50 g | 50 g | 50 g | 50 g | 50 g | 50 g |
| Phenoxyethanol | 4g | 4g | 4g | 4g | 4g | 4g | 4g | 4g | 4g |
| Ultrapure water | Bal. | Bal. | Bal. | Bal. | Bal. | Bal. | Bal. | Bal. | Bal. |
| Total | 1,000 g | 1,000 g | 1,000 g | 1,000 g | 1,000 g | 1,000 g | 1,000 g | 1,000 g | 1,000 g |

<Experiment 3-2: Preparation of Test Hair Bundles, and Evaluation of Feeling of Use of Hair-Setting Compositions Comprising an Isomaltooligosaccharide using Test Hair Bundles>

Test hair bundles were prepared in the same manner as in Experiment 1-3 except that Test Compositions 14 to 21 in liquid form comprising the isomaltooligosaccharide mixture and Control Composition 3 in liquid form not comprising the isomaltooligosaccharide mixture were used instead of Test Compositions 1 to 4 in liquid form comprising an isomaltooligosaccharide and Control Composition 1 in liquid form comprising no isomaltooligosaccharide. The feeling of use (film-forming texture, stickiness) and the property (flaking after combing) when the thus prepared compositions were applied to hair bundles were evaluated in the same manner as in Experiment 2-3. The obtained results were shown in Table 7.

[Table 7]

| | Concentration of Isomaltooligosaccharides in the composition (in terms of solid content) | Film-Forming Texture | Stickiness | Flaking after Combing | Overall Evaluation |
|---|---|---|---|---|---|
| Hair Bundle Applied with Control Composition 3 | 0% by mass | × | ◎ | △ | × |
| Hair Bundle Applied with Test Composition 14 | 0.1% by mass | × | ◎ | ○ | △ |
| Hair Bundle Applied with Test Composition 15 | 0.5% by mass | ○ | ◎ | ◎ | ○ |
| Hair Bundle Applied with Test Composition 16 | 1% by mass | ◎ | ◎ | ◎ | ◎ |
| Hair Bundle Applied with Test Composition 17 | 5% by mass | ◎ | ◎ | ◎ | ◎ |
| Hair Bundle Applied with Test Composition 18 | 10% by mass | ◎ | ◎ | ◎ | ◎ |

(continued)

|  | Concentration of Isomaltooligosaccharides in the composition (in terms of solid content) | Film-Forming Texture | Stickiness | Flaking after Combing | Overall Evaluation |
|---|---|---|---|---|---|
| Hair Bundle Applied with Test Composition 19 | 20% by mass | ◎ | ○ | ○ | ○ |
| Hair Bundle Applied with Test Composition 20 | 25% by mass | ○ | △ | △ | △ |
| Hair Bundle Applied with Test Composition 21 | 30% by mass | ○ | × | × | × |

As shown in Table 7, as a result of the evaluation using the isomaltooligosaccharide mixture prepared by mixing isomaltose and panose at the mass ratio of 1:3 as isomaltooligosaccharides and with the hair bundles applied with Control Composition 3 or Test Compositions 14 to 21 in liquid form prepared by varying the final concentration of the isomaltooligosaccharide in the test compositions in the range of 0 to 30% by mass, the feeling of use (film-forming texture) of the test composition was judged favorable (i.e., judged "O" or "◎") when the final concentration of the isomaltooligosaccharide in the test compositions was in the range of 0.5 to 30% by mass, and was judged very favorable (i.e., judged " ◎ ") when the final concentration of the isomaltooligosaccharide was in the range of 1 to 20% by mass. Meanwhile, the feeling of use (stickiiness) was judged favorable (i.e., judged "○" or "◎") when the final concentration of the isomaltooligosaccharide in the test compositions was in the range of 0 to 20% by mass, and judged very favorable (i.e., judged ◎") when the final concentration of the isomaltooligosaccharide was in the range of 0 to 10% by mass. The property (flaking after combing) was judged favorable (i.e., judged " ○ " or " ◎ ") when the final concentration of the isomaltooligosaccharide in the test compositions was in the range of 0.1 to 20% by mass, and judged very favorable (i.e., judged OO ") when the final concentration of the isomaltooligosaccharide was in the range of 0.5 to 10% by mass. Overall evaluation was judged favorable (i.e., judged "○" or "◎") when the final concentration of the isomaltooligosaccharide in the test compositions was in the range of 0.5 to 20% by mass, and judged very favorable (i.e., judged OO ") when the final concentration of the isomaltooligosaccharide was in the range of 1 to 10% by mass. The above results indicate that a hair-setting composition showing the good feeling of use and property may be obtained when the agent for enhancing a hair-setting ability comprising an isomaltooligosaccharide as an active ingredient was used in a hair-setting composition in liquid form at the final concentration in the range of 0.5 to 20% by mass with respect to the total mass of the hair-setting composition in liquid form, and that a hair-setting composition showing the excellent feeling of use and property may be obtained when the agent for enhancing a hair-setting ability comprising an isomaltooligosaccharide as an active ingredient is used at the final concentration in the range of 1 to 10% by mass.

<Experiment 4: Effect of Isomaltooligosaccharides on Hair-Setting Ability and Hair-Arranging Ability>

Hair-setting compositions comprising an isomaltooligosaccharide were prepared and their hair-setting ability and hair-arranging ability were evaluated.

<Experiment 4-1: Preparation of Test Compositions>

Test Compositions and Control Compositions used in this experiment were prepared in the following method.

"Control Composition 4"

Control Composition 4 was prepared in the same manner as Control Composition 1 in Experiment 1-2.

"Control Composition 5"

Control Composition 5 in a liquid composition form was prepared in the same manner as Control Composition 1 in Experiment 1-2 except that (methacryloyloxyethyl carboxybetaine/alkyl methacrylate) copolymer (Product Name "RAM Resin", Osaka Organic Chemical Industry Ltd., Hyogo, Japan) was used as a resin having a hair-setting ability instead of (vinylpyrrolidone/VA) copolymer in Control Composition 4.

"Control Composition 6"

Control Composition 6 in a liquid composition form was prepared in the same manner as Test Composition 1 in Experiment 1-2 except that maltitol (Product Name "Amalty 75%, Mitsubishi Corporation Life Sciences Limited, Tokyo, Japan) and PEG-6/PEG-32 (Product Name "PEG#1500", NOF CORPORATION, Tokyo, Japan), which were conventionally used as additives to enhance the hair-arranging ability (toughness) of hair bundles, were used instead of the isomaltooligosaccharide-containing saccharide solution prepared in Experiment 1-1.

"Control Composition 7"

Control Composition 7 in a liquid composition form was prepared in the same manner as Test Composition 1 in Experiment 1-2 except that (methacryloyloxyethyl carboxybetaine/alkyl methacrylate) copolymer was used instead of (vinylpyrrolidone/VA) copolymer and further that maltitol (Product Name "Amalty 75%, Mitsubishi Corporation Life Sciences Limited, Tokyo, Japan) and PEG-6/PEG-32 (Product Name "PEG#1500", NOF CORPORATION, Tokyo, Japan) were used instead of the isomaltooligosaccharide-containing saccharide solution prepared in Experiment 1-1.

"Test Compositions 22 and 23"

Test composition 22 in a liquid composition form was prepared in the same manner as Test Composition 1 in Experiment 1-2 except that (methacryloyloxyethyl carboxybetaine/alkyl methacrylate) copolymer was used instead of (vinylpyrrolidone/VA) copolymer. On the other hand, Test Composition 23 in a liquid composition form was prepared in the same manner as Test Composition 22 except that the formulation amount of the isomaltooligosaccharide-containing saccharide solution prepared in Experiment 1-1 was reduced to half.
In addition to Control Compositions 4 to 7 and Test Compositions 22 and 23 prepared as described above, Test composition 1 prepared in Experiment 1-2 was used in this experiment. The compositions of Test Compositions 1, 22, and 23, and Control Compositions 4 to 7 were shown in Table 8.

Table 8]

| | Control Composition | | | | Test Composition | | |
|---|---|---|---|---|---|---|---|
| | 4 | 5 | 6 | 7 | 1 | 22 | 23 |
| Isomaltooligosaccharide-containing saccharide solution prepared in Experiment 1-1 (in terms of solid) | - | - | - | - | 100 g | 100 g | 50 g |
| Ethanol | 100 g | 100 g | 100 g | 100 g | 100 g | 100 g | 100 g |
| (vinylpyrrolidone/VA) copolymer | 50 g | - | 50 g | - | 50 g | - | - |
| (Methacryloyloxyethyl carboxybetaine/alkyl methacrylate) copolymer/ethanol (Effective Amount 18%) | - | 50 g | - | 50 g | - | 50 g | 50 g |
| Maltitol | - | - | 50 g | 50 g | - | - | - |
| PEG-6/PEG-32 | - | - | 50 g | 50 g | - | - | - |
| Phenoxyethanol | 4g | 4g | 4g | 4g | 4g | 4g | 4g |
| Ultrapure water | Bal. | Bal. | Bal. | Bal. | Bal. | Bal. | Bal. |
| Total | 1,000 g | 1,000 g | 1,000 g | 1,000 g | 1,000 g | 1,000 g | 1,000 g |

<Experiment 4-2: Preparation of Test Hair Bundles for Measurement of Hair-Setting Ability and Hair-Arranging Ability and Evaluation of Hair-Setting Ability>

Test Hair Bundles were prepared in the same manner as in Experiment 1-3 except that Test Compositions 1, 22 and 23 or Control Compositions 4 to 7 were applied instead of Test Compositions 2 to 4 and Control Composition 1.

For each test hair bundle, the hair-setting ability was measured following the method according to Experiment 1-4. Table 9 shows mean and standard deviation of the maximum load when the hair bundle was broken (the bending strength) which was measured for the hair bundles applied with Test Compositions or Control Compositions.

<Experiment 4-3: Evaluation of Hair-Arranging Ability>

Hair-arranging ability was evaluated using the toughness of hair bundles applied with a test composition or a control composition (hereinafter also referred to as "Test Composition-applied Hair Bundle" or "Control Composition-applied Hair Bundle", respectively) as an indicator. The toughness of hair bundle was measured using a rheometer (Product Name "CR-500DX-SII", Sun Scientific Co., Ltd., Tokyo, Japan). In detail, the Test Composition-applied Hair Bundles and Control Composition-applied Hair Bundles were extended over two measurement tables with a width of 1 cm and a height of 5 cm and having horizontal top surfaces, which were set at an interval of 7.8 cm. To the extended hair bundles, the pressure was applied using a metal probe with a wedge-shaped jig (Product Name: "Pressure Sensitive Axis No. 34 Tooth Type (A)", Sun Scientific Co., Ltd., Tokyo Japan) and lowering the probe vertically at a speed of 60 mm/min. After the probe contacted the hair bundle, the probe was lowered for 3 cm. During this time, the load was measured every 50 msec. The toughness of the hair bundle was evaluated using the migration distance of the probe as an indicator. Herein, the migration distance of the probe is the distance travelled by the probe and the migration distance from the time when the load indicates the half value of the maximum load after the probe touches the hair bundle and before the load reaches the maximum load to the time when half the maximum load is indicated again after the load reaches the maximum load. Measurements of the toughness of the hair bundles applied with Test Compositions or Control Compositions were performed three times using three similarly prepared hair bundles. Table 9 shows mean and standard deviation of the migration distance between the two halfvalues of the maximum load when hair bundles were broken, which was measured for each of the hair bundles applied with Test Compositions or Control Compositions.

[Table 9]

|  | Bending Strength (N) | Migration Distance (cm) |
|---|---|---|
| Control Composition 4 | 0.34 ± 0.09 | 9.33 ± 2.83 |
| Control Composition 5 | 0.35 ± 0.07 | 8.48 ± 0.93 |
| Control Composition 6 | 0.17 ± 0.04 | 17.12 ± 2.41 |
| Control Composition 7 | 0.13 ± 0.03 | 10.95 ± 4.49 |
| Test Composition 1 | 0.59 ± 0.16 | 17.63 ± 5.29 |
| Test Composition 22 | 0.52 ± 0.08 | 20.77 ± 2.84 |
| Test Composition 23 | 0.35 ± 0.13 | 25.03±4.37 |

The hair-setting ability and the hair-arranging ability were evaluated using the hair bundles applied with either one of Test Compositions 1, 22, 23 and Control Compositions 4 to 7. As shown in Table 9, for the hair bundles applied with Control Compositions 4 and 5, which only contained a resin for hair-setting, the bending strengths, an indicator of the hair-setting ability, were 0.34±0.09 N and 0.35±0.07 N, respectively, and the migration distances, an indicator of the hair-arranging ability, were 9.33±2.83 cm and 8.48±0.93 cm, respectively. In contrast, for the hair bundles applied with Control Compositions 6 and 7, which contained a conventional additive used for purpose of enhancing the toughness of hair bundles, the migration distances were 17.12±2.41 cm and 10.95±4.49 cm, respectively, wherein increase in the migration distance compared to the hair bundles applied with Control Compositions 4 and 5 was observed. Meanwhile, the bending strengths were 0.17 ± 0.04 N and 0.13 ± 0.03 N, respectively, wherein decrease in the bending strength compared to the hair bundles applied with Control Compositions 4 and 5 was observed. This result indicates that conventional additives such as maltitol and PEG-6/PEG-32 can enhance the hair-arranging ability (the toughness) but decreases the hair-setting ability (the hardness). In sharp contrast, for the hair bundles applied with Test Compositions 1 and 22 containing the isomaltooligosaccharide-containing saccharide solution prepared in Experiment 1-1 as isomaltooligosaccharide, the migration distances were 17.63±5.29 cm and 20.77±2.84 cm, respectively, which were comparable or greater than those of the hair bundles applied with Control Compositions 5 and 6 containing the conventional additives, and in addition the bending strengths were 0.59±0.16N and 0.52±0.08N, which were much greater than those of the hair bundles applied with Control Compositions 5 and 6 containing the conventional additives. This result indicates that the isomaltoligosaccharidecontaining saccharide shows the effect of enhancing both the hair-setting ability and the hair-arranging ability. On the other hand, the bending strength and the migration distance of the hair bundle applied with Test Composition 23, in which the amount of the isomaltooligosaccharide-containing saccharide solution prepared in Experiment 1-1 was halved, was 0.35±0.13N and 25.03±4.37cm, respectively, which were slightly inferior to the hair setting ability of Test Compositions 1 and 22. Nevertheless, it was shown that Test Composition 23 improved the hair arranging ability while maintaining the hair setting ability.

The above result shows that isomaltooligosaccharides have the effect of imparting not only the hair-setting ability but also the hair-arranging ability, in sharp contrast to additives conventionally used in the art. Therefore, isomaltooligosaccharides may be advantageously used as an excellent styling material for enhancing both of the hair-setting ability and the hair-arranging ability.

<Experiment 5: Evaluation of Feel and Properties of Hair Applied with Gel Composition Containing Isomaltooligosaccharide>

In order to examine, in more detail, the feeling of use of a hair-setting composition containing isomaltooligosaccharide, a gel composition containing isomaltooligosaccharide was prepared. The gel composition was applied to hair, and the feeling (tangling, smoothness, and stickiness) of the hair was evaluated, followed by evaluation of the property (flaking).

<Experiment 5-1: Preparation of Test Composition in Gel Form>

A solution containing 2% by mass of carbomer was prepared by adding 7 g of carbomer (Product Name "Carbopol 980", Lubrizol Corporation, OH, USA) to ultrapure water, followed by stirring using a stirrer to uniformity. As an isomaltooligosaccharide, 30 g or 50 g of an isomaltooligosaccharide-containing saccharide (Product Name "Lissenare" (Registered Trademark); Solid content: 74% by mass or more; Isomaltooligosaccharide content per solid: 50% by mass or more; Panose content per solid: 28% by mass or more; Hayashibara Co., Ltd., Okayama, Japan) was dissolved in ultrapure water, followed by stirring using a stirrer to uniformity. To the obtained mixture, the solution containing 2% by mass of carbomer was added as a thickener, and the mixture was stirred using a stirrer. After stirring, 3 g of potassium hydroxide was further added and the mixture was mixed using a glass rod to obtain solutions containing 3% by mass or 5% by mass of the isomaltooligosaccharide. On the other hand, to 200 g ethanol, 60 g of (vinylpyrrolidone/VA) copolymer (Product Name "Luvisko VA64P", BASF Japan, Ltd.) was added as a resin having a hair-setting effect, and 4 g of phenoxyethanol was further added as a preservative. They were made dissolved using a stirrer to obtain a (vinylpyrrolidone/VA) copolymer-containing solution. Each of the solution containing 3% by mass or 5% by mass of the isomaltooligosaccharide was mixed with the (vinylpyrrolidone/VA) copolymer-containing solution using a glass rod to uniformity to obtain Test Gel 1 and Test Gel 2, respectively. Meanwhile, as a Control Gel, a gel composition was prepared in the same manner as Test Gel 1 except that 20 g of glycerin and 10 g of sorbitol, which were conventionally used as moisturizers, were used instead of 30 g of the isomaltooligosaccharide-containing saccharide. The compositions of Test Gel 1 and 2 as well as Control Gel were shown in Table 10.

[Table 10]

|  | Control Gel | Test Gel 1 | Test Gel 2 |
|---|---|---|---|
| Isomaltooligosaccharide-containing saccharide | - | 30 g | 50 g |
| Ethanol | 200 g | 200 g | 200 g |
| (vinylpyrrolidone/VA) copolymer | 60 g | 60 g | 60 g |
| Glycerin | 20 g | - | - |
| Sorbitol | 10 g | - | - |
| Carbomer | 7g | 7 g | 7 g |
| Potassium hydroxide | 3 g | 3 g | 3 g |
| Phenoxyethanol | 4 g | 4 g | 4 g |
| Ultrapure water | Bal. | Bal. | Bal. |
| Total | 1,000 g | 1,000 g | 1,000 g |

<Experiment 5-2: Evaluation of Feel of Hair Applied with Gel Compositions Containing Isomaltooligosaccharide>

The feel of hair was evaluated as to the hair on which Test Gel 1, 2 or Control Gel obtained in Experiment 5-1 was applied.

The feel of hair was evaluated based on the frictional force of the hair on which each test gel or control gel was applied.

The frictional force of the hair was measured using a sliding table type friction evaluation meter (Product name "Tribo-master TL201Ts", Trinity-Lab Inc., Tokyo, Japan). Briefly, twenty pieces of 5 cm-long hairs on which any gel was not applied were pasted on a 7.6 cm wide, 2.6 cm long slide glass at intervals of 1 mm to prepare a hair-pasted slide glass, which was fixed to the sliding table type friction evaluation meter. The tactile contactor was lowered and placed on the end of the fixed hair and was moved 4 times against the surface of the hair at a load of 50 g, a speed of 2 mm/sec, and a distance of 20 mm, and the maximum friction coefficient ($\mu$S), the mean dynamic friction coefficient (MIU), and the mean deviation of dynamic friction coefficient (MMD) at this time were measured. After measurement, the tactile contactor was once removed from the friction evaluation meter. Then, to the central portion of the tactile contactor, 5 $\mu$L of Test Gel 1 or 2, or Control Gel was applied, and the tactile contactor was attached back to the friction evaluation meter. The tactile contactor was lowered and placed on the end of the hair-pasted slide glass and was moved under the same conditions as when the measurement for the hairs on which any gel was not applied was carried out, and the maximum friction coefficient ($\mu$S), the mean dynamic friction coefficient (MIU), and the mean deviation of dynamic friction coefficient (MMD) after 0, 1, 3, 5, 7, and 10 minutes were evaluated. For each gel composition, measurements were carried out 4 times using 4 hair-pasted slide glasses.

<Experiment 5-3: Result of Evaluation of the Feel of the Hair>

The measurement results of the frictional force of the hair when Test Gel 1 or 2 or Control Gel was applied measured in Experiment 5-2 are interpreted as evaluating following three items of the feel: "tangling", "smoothness", and "stickiness". "Tangling" is evaluated based on the maximum friction coefficient ($\mu$S), which shows the feeling of resistance at the moment of starting stroking hair. In other words, a large value of the maximum friction coefficient ($\mu$S) indicates that the feeling of resistance is large when the gel composition is applied to hair and a finger starts moving. "Smoothness" is evaluated based on the mean dynamic friction coefficient (MIU), which shows the feeling of resistance when running fingers through hair. In the other words, a large value of the mean dynamic friction coefficient (MIU) indicates that fingers cannot run through hair smoothly and it is difficult to move fingers through hair. "Stickiness" is evaluated based on the mean deviation of dynamic friction coefficient (MMD). A large value of the mean deviation of dynamic friction coefficient (MMD) indicates that the stick-slip phenomenon, in which fingers are repeatedly caught by or slipped on hair when sliding through the hair, is likely to occur and that hair is sticky. From the mean values of the maximum friction coefficient ($\mu$S), mean dynamic friction coefficient (MIU), and mean deviation of dynamic friction coefficient (MMD) obtained for the hairs on which Test Gel 1 or 2, or Control Gel was applied, each corresponding value measured for the hairs on which any gel was not applied was subtracted to obtain $\Delta$ value as an indicator for each evaluation item of the feel. Mean and standard deviation of the $\Delta$ value for the maximum friction coefficient ($\Delta\mu$S), dynamic friction coefficient ($\Delta$MIU), and mean deviation of dynamic friction coefficient ($\Delta$MMD) were shown in Tables 11, 12, and 13, respectively.

<Statistical Analysis>

Measured values were expressed as mean $\pm$ standard deviation (SD). Statistical analysis was performed by unpaired t-test. A significance level (p value) of less than 0.05 (less than 5% risk) was judged to be significant.

[Table 11]

| | Maximum Friction Coefficient ($\Delta\mu$S) | | | | | |
|---|---|---|---|---|---|---|
| Measurement Time (min) | 0 | 1 | 3 | 5 | 7 | 10 |
| Control Gel | -0.055 ±0.047 | 0.108 ±0.044 | 0.337 ±0.088 | 1.008 ±0.314 | 0.775 ±0.362 | 0.706 ±0.109 |
| Test Gel 1 | 0.067 ±0.144 | 0.094 ±0.117 | 0.251 ±0.205 | 0.281 ±0.212** | 0.180 ±0.106** | 0.210 ±0.176** |
| Test Gel 2 | -0.026 | 0.039 | 0.212 | 0.157 | 0.115 | 0.131 |

(continued)

| | Maximum Friction Coefficient ($\Delta\mu S$) | | | | | |
|---|---|---|---|---|---|---|
| Measurement Time (min) | 0 | 1 | 3 | 5 | 7 | 10 |
| | ±0.052 | ±0.040* | ±0.100 | ±0.120** | ±0.017** | ±0.029** |
| * : p<0.05 vs the hairs on which Control Gel was applied<br>** : p<0.01 vs the hairs on which Control Gel was applied | | | | | | |

The friction forces of the hairs applied with Test Gels 1 and 2 containing isomaltooligosaccharides were evaluated. As shown in Table 11, at 5 minutes after application, the Δ value of the maximum friction coefficient of the hairs applied with Control Gel, which was prepared in the same manner as Test Gel 1 except that glycerin and sorbitol were incorporated instead of isomaltooligosaccharides, was 1.008±0.314, while the Δ values of the maximum friction coefficient of the hairs applied with Test Gels 1 and 2 were 0.281 ±0.212 and 0.157±0.120, respectively, which were revealed to be remarkably smaller than the Δ value of the maximum friction coefficient of the hairs applied with Control Gel. At 10 minutes after application when the applied gel was thought to be completely dried, the Δ value of the maximum friction coefficient of the hairs applied with Control Gel was as high as 0.706±0.109, while the Δ values of the maximum friction coefficient of the hairs applied with Test Gel 1 and 2 remained to be as low as 0.210 ± 0.176 and 0.131 ± 0.029, respectively, which were revealed to be remarkably smaller than the Δ value of the maximum friction coefficient of the hairs applied with Control Gel. This result indicates that the Δ value of the maximum friction coefficient is remarkably reduced in the hairs applied with Test Gels containing isomaltooligosaccharides compared to the hairs applied with Control Gels and the feeling of hairs with less "tangling" can be obtained.

[Table 12]

| | Mean Dynamic Friction Coefficient ($\Delta$MIU) | | | | | |
|---|---|---|---|---|---|---|
| Measurement Time (min) | 0 | 1 | 3 | 5 | 7 | 10 |
| Control Gel | 0.022 ±0.115 | 0.123 ±0.071 | 0.279 ±0.080 | 0.261 ±0.029 | 0.163 ±0.073 | 0.178 ±0.117 |
| Test Gel 1 | 0.134 ±0.118 | 0.078 ±0.056 | 0.201 ±0.057 | 0.156 ±0.100** | 0.084 ±0.083 | 0.078 ±0.019 |
| Test Gel 2 | 0.044 ±0.037 | 0.064 ±0.011* | 0.111 ±0.026** | -0.001 ±0.031** | 0.032 ±0.030** | 0.029± 0.030* |
| * : p<0.05 vs the hairs on which Control Gel was applied<br>** : p<0.01 vs the hairs on which Control Gel was applied | | | | | | |

As shown in Tables 12, at 3 minutes after application, the Δ value of the mean dynamic friction coefficient of the hairs applied with Control Gel was 0.279±0.080, while that of the hairs applied with Test Gel 1 was as small as 0.202±0.057 and that of the hairs applied with Test Gel 2 was even smaller 0.112±0.026. Similarly, at 5 minutes after application, the Δ value of the mean dynamic friction coefficient of the hairs applied with Control Gel was 0.261±0.029, while that of the hairs applied with Test Gel 1 was as small as 0.156±0.100 and that of the hairs applied with Test Gel 2 was even smaller -0.001±0.031. Furthermore, at 10 minutes after application when the applied gel was thought to be completely dried, the Δ value of the mean dynamic friction coefficient of the hairs applied with Control Gel was 0.178±0.117, while that of the hairs applied with Test Gel 1 was as small as 0.078±0.019, and that of the hairs applied with Test Gel 2 was even smaller 0.029±0.030. This result indicates that the Δ value of the mean dynamic friction coefficient is remarkably reduced in the hairs applied with Test Gels containing isomaltooligosaccharides compared to the hairs applied with Control Gels and the feeling of hairs with more "smoothness" can be obtained. In particular, it was shown based on the remarkably decreased Δ value of the mean dynamic friction coefficient that particularly "smooth" feeling can be obtained when Test Gel 2 containing 5% by mass of isomaltooligosaccharides is used.

[Table 13]

| | Mean Deviation of Dynamic Friction Coefficient (ΔMMD) | | | | | |
|---|---|---|---|---|---|---|
| Measurement Time (min) | 0 | 1 | 3 | 5 | 7 | 10 |
| Control Gel | 0.005 ±0.002 | 0.041 ±0.011 | 0.085 ±0.038 | 0.100 ±0.032 | 0.101 ±0.042 | 0.081 ±0.047 |
| Test Gel 1 | 0.003 ±0.003 | 0.012 ±0.007** | 0.034 ±0.010* | 0.044 ±0.036* | 0.011 ±0.004** | 0.009 ±0.005* |
| Test Gel 2 | 0.003 ±0.001 | 0.020 ±0.014* | 0.011 ±0.002** | 0.082 ±0.025 | 0.060 ±0.036 | 0.050 ±0.045 |
| * : p<0.05 vs the hairs on which Control Gel was applied<br>** : p<0.01 vs the hairs on which Control Gel was applied | | | | | | |

As shown in Table 13, at 3 minutes after application, the Δ value of the mean deviation of dynamic friction coefficient of the hairs applied with Control Gel was $0.085 \pm 0.038$, while that of the hairs applied with Test Gels 1 and 2 were $0.034 \pm 0.010$ and $0.011 \pm 0.002$, respectively, which were revealed to be remarkably smaller than that of the hairs applied with Control Gels. Meanwhile, at 10 minutes after application when the applied gel was thought to be completely dried, the Δ value of the mean deviation of dynamic friction coefficient of the hairs applied with Control Gel was $0.081 \pm 0.047$, while that of the hairs applied with Test Gel 1 was remarkably smaller $0.0094 \pm 0.005$ and that of the hairs applied with Test Gel 2 was also as small as $0.050 \pm 0.045$. This result indicates that the Δ value of the mean deviation of dynamic friction coefficient is remarkably reduced in the hairs applied with Test Gels containing isomaltooligosaccharides compared to the hairs applied with Control Gels and the feeling of hair with less "Stickiness" can be obtained.

As shown above, the results shown in Tables 11 to 13 indicate that the gel compositions containing isomaltooligosaccharides are not only good at the hair-setting ability but also useful for finishing the feeling of the set hair better compared to the gel composition containing glycerin and sorbitol, which are conventionally used as moisturizers.

<Experiment 5-4: Evaluation of Properties of Hairs Applied with Gel-Type Composition Containing Isomaltooligosaccharides>

Next, the properties of the hairs applied with Test Gel 2 and Control Gel, obtained in Experiment 5-1, were evaluated. As the properties of the hairs, the effect of isomaltooligosaccharides on the flaking after combing was evaluated. Herein, the term "flaking" refers to a state in which a resin for setting hair comprised in hair gels, etc., gets dried and solidified and appears on the surface of the hairs in a dusty manner. Conventionally, moisturizers such as glycerin and sorbitol, etc. are added for purpose of reducing flaking.

<Experiment 5-5: Preparation of Test Hair Bundles for Evaluating the Effect on Flaking>

To the hair bundles prepared in Experiment 1-3, 0.8 g of Test Gel 2 was dropped and evenly applied by hand. After application of Test Gel 2, the hair bundles were incubated in a constant temperature and humidity chamber (22°C, relative humidity 50%) for 1 hour and used for evaluating the effect on flaking. The hair bundles applied with Control Gel were prepared in the same manner except that Control Gel was applied instead of Test Gel 2.

<Experiment 5-6: Evaluation of Flaking>

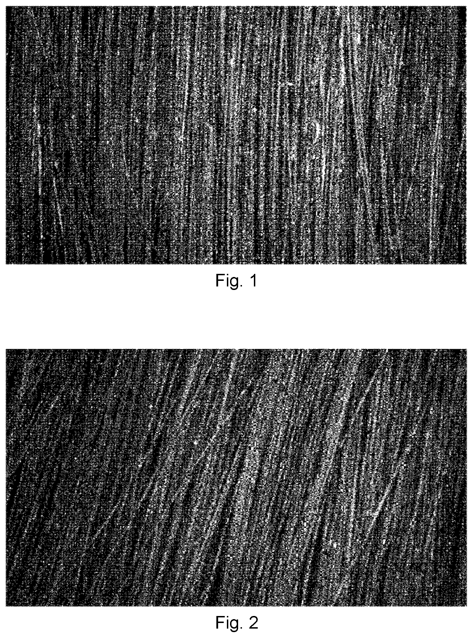

The flaking was visually evaluated based on the amount of resin wastes, which were contained in Test Gel 2 and peeled off by combing, as an indicator. In detail, the hair bundles applied with Test Gel 2 and those applied with Control Gel, prepared in Experiment 5-5, were combed 5 times and subsequently the flaking state was visually observed and photographed. The photographs showing the flaking state after combing of the hair bundles applied with Control Gel or Test Get 2 were shown in Figure 1 and Figure 2, respectively.

As shown in Figures 1 and 2, much flaking was observed in the hair bundles applied with Control Gel, whereas little flaking was observed in the hair bundles applied with Test Gel 2. This result indicates that isomaltooligosaccharides have the better effect of reducing flaking compared to sorbitol and glycerin, which are conventionally used as moisturizers for purpose of reducing flaking.

The above result indicates that the gel composition containing an isomaltooligosaccharide has not only the effect of finishing the feeling of set hair better but also the effect of reducing flaking after combing. An isomaltooligosaccharide may be advantageously used as an excellent styling material that improves finishing of set hair.

<Experiment 6: Effect of Formulating Ratio of Isomaltose and Panose on Treatment Effect>

In order to examine the effect of the formulating ratio of isomaltose and panose on the treatment effect, using isomaltose and panose as isomaltooligosaccharides, test compositions containing the two isomaltooligosaccharides at different formulating ratios were prepared and the treatment effect on damaged hairs treated with the test compositions was evaluated.

<Experiment 6-1: Preparation of Test Compositions>

Test Compositions 24 to 30 containing isomaltose and panose as isomaltooligosaccharides at the formulating ratios of 1:0, 3:1, 1:1, 1:3, 1:10, 1:15, and 0:1 were prepared. Briefly, an aqueous solution containing 20% by mass of isomaltose and an aqueous solution containing 20% by mass of panose were prepared, and the two solutions were mixed at the mass ratio shown in Table 14 to obtain five isomaltooligosaccharide mixtures with different mixing ratios of isomaltose and panose. The obtained isomaltooligosaccharide mixtures were dissolved in ultrapure water at the final concentration of the isomaltooligosaccharide mixture of 5% by mass, followed by stirring and mixing using a vertex mixer to obtain Test Compositions 24 to 30 in liquid form. The compositions of Test Compositions 24 to 30 were shown in Table 14.

[Table 14]

| | Test Compositions | | | | | | |
|---|---|---|---|---|---|---|---|
| | 24 | 25 | 26 | 27 | 28 | 29 | 30 |
| 20% isomaltose solution | 2.50 g | 1.88 g | 1.25 g | 0.63 g | 0.23 g | 0.16 g | - |
| 20% panose solution | - | 0.62 g | 1.25 g | 1.87 g | 2.27 g | 2.34 g | 2.50 g |
| Ultrapure water | Bal. | Bal. | Bal. | Bal. | Bal. | Bal. | Bal. |
| Total | 10g | 10g | 10g | 10g | 10g | 10g | 10g |

<Experiment 6-2: Preparation of Test Hair Bundles (Healthy Hair, Damaged Hair, and Test Composition-Treated Hair Bundle) for Measuring Damages on Hair Surface>

The healthy hair, damaged hair, and hair bundles treated with Test Compositions 24 to 30 (hereinafter also referred to as "Test Composition-treated Hair Bundles") used in this experiment were prepared in the following method.

"Preparation of Healthy Hair"

Hair strands (Product Name "Human black hair BS-PG"; Beaulax Co., Ltd., Saitama, Japan) were aligned at the roots to a length of 15 cm to obtain a hair bundle, and 1 g of the hair bundle was immersed in an aqueous solution containing 2.7% by mass of sodium laureth sulfate (prepared by diluting sodium laureth sulfate (Product Name "EMAL E-27C", Kao Corporation, Tokyo Japan) 10-fold with ultrapure water) and incubated for 60 mins at 25°C.After incubation, the hair bundle was rinsed thoroughly under running tap water, and drained with paper towels. The hair bundle was then rinsed twice with ultrapure water and drained with paper towels again. Thereafter, the hair bundle was dried with cool air and dried overnight or longer in an environment with a temperature of 22°C and a relative humidity of 50% to obtain a healthy hair.

"Preparation of Damaged Hair"

A mixed solution (pH 10.5 to 11.0; hereinafter referred to as "bleaching solution") of 1.4% by mass of ammonia solution and 3% by mass of hydrogen peroxide solution was prepared. The prepared healthy hair was immersed in the bleaching solution and incubated for 60 mins at 30°C. After incubation, the hair bundle was rinsed thoroughly under running tap water, and drained with paper towels. The hair bundle was then rinsed twice with ultrapure water and drained with paper towels again. Thereafter, the hair bundle was dried with cool air and dried overnight or longer in an environment with a temperature of 22°C and a relative humidity of 50%. Damaged hairs were obtained by repeatedly subjecting the healthy hair to a series of treatments from immersion in the bleach solution, rinsing with ultrapure water, to drying under a constant environment, for three times.

"Preparation of Test Composition-Treated Hair Bundle"

A hair bundle consisting of 1 g of the prepared damaged hair was immersed in the test compositions prepared in Experiment 6-1 and incubated for 10 mins at 30°C. After incubation, the hair bundle was drained with paper towels and then rinsed with ultrapure water under shaking for 10 seconds. The hair bundle was drained with paper towels again, dried with cool air, and dried overnight or longer in an environment with a temperature of 22°C and a relative humidity of 50% to obtain a Test Composition-treated hair bundle.

<Experiment 6-3: Evaluation of Hair Treatment Effect in Test Composition-Treated Hair Bundle Treated with Test Compositions in Liquid Form Containing Isomatooligosaccharide Mixture>

To examine the hair treatment effect of the test compositions containing the isomaltooligosaccharide mixture, texture of the hair bundles treated with the test compositions prepared in Experiment 6-2 was evaluated. In this experiment, the hair treatment effect was evaluated by 3 trained panelists using the texture (suppleness, smoothness, silkiness, and luster) of the hair bundles when touching the test composition-treated hair bundles with bare hands as an indicator.

The evaluation was made on the following fours items: "Suppleness", "Smoothness", "Silkiness" and "Luster". "Suppleness" refers to the degree of flexibility that allows hair to bend without stiff feeling when touching the hair. In this experiment, the degree of flexibility that allows hair to bend without stiff feeling when touching the hair was evaluated using the damaged hair that had not been treated with the test composition as a standard. "Smoothness" refers to the absence of resistance feeling when touching the hair. In this experiment, the degree of slipperiness when touching the hair was evaluated using the damaged hair that had not been treated with the test composition as a standard. "Silkiness" refers that the hair is unentangled without being tangled when touching the hair. In this experiment, the ease of finger combing when touching the hair was evaluated using the damaged hair that had not been treated with the test composition as a standard. As for "Luster", the degree of luster seen when the hair bundles were aligned was visually evaluated, using the damaged hair that had not been treated with the test composition as a standard.

Evaluation for each item was carried out on a 3-point scale based on the criteria of "2: Improved", "1: Slightly Improved", "0: Not Changed". The average point was calculated by dividing the sum of the points of all panelists by the number of panelists. It was judged "◎" when the average point was 1 point or more, "○" when the average point was 0.5 points or more and less than 1 point, "△" when the average point was more than 0 point but less than 0.5 points, and "×" when the average point was 0 point. The obtained results were shown in Table 15, together with the average area ratio calculated in Experiment 6-3.

[Table 15]

| | Mass ratio of isomaltose : panose in test composition (in terms of solid content) | Suppleness | Smoothness | Silkiness | Luster | Overall Evaluation |
|---|---|---|---|---|---|---|
| Healthy Hair | - | - | - | - | - | - |
| Damaged Hair | - | - | - | - | - | - |
| Hair Bundle Treated with Test Composition 24 | 1:0 | △ | × | × | × | △ |
| Hair Bundle Treated with Test Composition 25 | 3:1 | △ | ◎ | × | △ | △ |
| Hair Bundle Treated with Test Composition 26 | 1:1 | △ | ◎ | ◎ | ◎ | ○ |

(continued)

|  | Mass ratio of isomaltose : panose in test composition (in terms of solid content) | Suppleness | Smoothness | Silkiness | Luster | Overall Evaluation |
|---|---|---|---|---|---|---|
| Hair Bundle Treated with Test Composition 27 | 1:3 | ◎ | ◎ | ◎ | ◎ | ◎ |
| Hair Bundle Treated with Test Composition 28 | 1:10 | ○ | ◎ | ◎ | ○ | ◎ |
| Hair Bundle Treated with Test Composition 29 | 1:15 | △ | × | △ | △ | △ |
| Hair Bundle Treated with Test Composition 30 | 0:1 | × | × | △ | ○ | △ |

As shown in Table 15, "suppleness" was judged favorable (i.e., judged "◎" or"○") when the mass ratio of isomaltose and panose was in the range of 1:3 to 1:10. "Smoothness" was judged very favorable (i.e., judged "◎") when the mass ratio of isomaltose and panose was in the range of 3:1 to 1:10. "Silkiness" was judged very favorable (i.e., judged "◎") when the mass ratio of isomaltose and panose was in the range of 1:1 to 1:10. And, "Luster" was judged favorable (i.e., judged "◎" or"○") when the mass ratio of isomaltose and panose was in the range of 1:1 to 1:10. This result indicates that when the mass ratio of isomaltose and panose is in the range of 1:1 to 1:10, in particular when the mass ratio of isomaltose and panose is in the range of 1:3 to 1:10, the excellent hair treatment effect is shown and the favorable texture is imparted to the hairs.

<Experiment 6-4: Measurement of Damages on Hair Surface>

In order to examine the effect of combination of isomaltose and panose on the hair treatment effect from another aspect, the effect of Test Composition 24, Test Composition 27 and Test Composition 30, containing isomaltose and panose at the mass ratios of 1:0, 1:3, and 0:1, respectively, on damages on the hair surface was evaluated.

Damage on the hair surface was evaluated using the fluorescence intensity of Rhodamine B (sold by FUJIFILM Wako Pure Chemical Corporation, Osaka, Japan), a cationic fluorescent reagent, as an indicator. The Rhodamine B staining method is a method for evaluating damage on the hair surface by using the distribution of Rhodamine B, a cationic fluorescent reagent, adsorbed on the hair surface with exposed anionic functional groups due to damages. The healthy hairs, damaged hairs, and the hair bundles treated with Test Composition 24, Test Composition 27, or Test Composition 30 (hereinafter also referred to as "Test Composition-treated Hair Bundle"), prepared in Experiment 6-2, were immersed in an aqueous solution containing 0.05% of Rhodamine B for 3 mins. After immersion, the hair bundles were washed with ultrapure water three times, drained with paper towels, and dried sufficiently. Thereafter, twenty hairs were taken out from each of the healthy hair, the damaged hair, and the Test Composition-treated hair bundles and were pasted on a slide glass with double-sided tape. Images of the pasted hairs were taken with a fluorescence microscope (Product Name "BX53", sold by Olympus Corporation, Tokyo, Japan) (Excitation wavelength: 530 to 550 nm, Fluorescence wavelength: 575 nm or longer) with an exposure time of 10 msec. Using an image analysis software (Product Name "cellSens", sold by Olympus Corporation, Tokyo, Japan), the obtained images were analyzed, and the ratio of the area of the hair surface showing a fluorescence intensity of 200 or more to the entire area of the hair surface (hereinafter simply referred to as "area ratio") was calculated and used as an indicator for damage on the hair surface. The damage on the hair surface was measured using 20 hairs taken from each of the healthy hair, the damaged hair, and the test composition-treated hair bundles. Mean and standard deviation of the area ratio obtained for each of the healthy hair, the damaged hair, and the test composition-treated hair bundles were shown in Table 16.

[Table 16]

| | Mass ratio of isomaltose : panose in test composition (in terms of solid content) | Area Ratio (%) |
|---|---|---|
| Healthy Hair | - | 0±0.0 |
| Damaged Hair | - | 81.5±19.1 |
| Hair Bundle Treated with Test Composition 24 | 1:0 | 87.5±16.6 |
| Hair Bundle Treated with Test Composition 27 | 1:3 | 41.3±30.7 |
| Hair Bundle Treated with Test Composition 30 | 0:1 | 61.8±19.1 |

As shown in Table 16, the area ratio for the healthy hair was 0±0.0%, whereas that of the damaged hair was 81.5±19.1%, indicating that the area ratio was increased in the damaged hair, in other words, that there were damages on the hair surface of the damaged hair. In contrast, in the hair bundles obtained by treating the damaged hair with Test Composition 24 containing only isomaltose as an isomaltooligosaccharide, the area ratio was 87.5±16.6%, indicating that the degree of the damage remained comparable to that of the damaged hair. On the other hand, in the hair bundles obtained by treating the damaged hair with Test Composition 30 containing only panose as an isomaltooligosaccharide, the area ratio was 61.8±19.1% and the decrease of the area ratio compared to the damaged hair was observed. In the hair bundles obtained by treating the damaged hair with Test Composition 27 containing isomaltose and panose at the mass ratio of 1:3, the area ratio was 41.3±30.7%, wherein the remarkable decrease of the area ratio compared to the damaged hair was observed. This result indicates that, when isomaltose and panose are used in combination as isomaltooligosaccharides, the particularly good hair treatment effect can be obtained also from the aspect of damages on the hair surface.

As shown above, based on the results shown in Table 15 and Table 16, it was shown that an isomaltooligosaccharide not only enhances the hair-setting ability but also is useful as a hair treatment ingredient for hair protection. The hair treatment effect is particularly remarkably shown when the two kinds of isomaltooligosaccharides, i.e., isomaltose and panose, are used in combination. It was shown that the effect of showing an excellent hair treatment effect is particularly remarkably exhibited when the mass ratio of isomaltose and panose is in the range of 1:1 to 1:10, and especially when the mass ratio of isomaltose and panose is in the range of 1:3 to 1:10.

<Experiment 7: Effect of Isomaltooligosaccharide Content on Hair Treatment Effect and Feeling of Use of Hair Treatment Compositions>

While keeping the formulating ratio (mass ratio) of isomaltose and panose fixed, hair treatment compositions which deferred from one another only in the varying isomaltooligosaccharide contents were prepared. Their feeling of use and the hair treatment effect during the process of from applying each composition to hair, washing it off, to drying the hair were evaluated.

<Experiment 7-1: Preparation of Test Emulsion Composition>

Forty five (45) grams of ultrapure water were put in a beaker, heated to 80°C using a hot water bath, and was added with 0.68 g of citric acid (Product Name "Purified citric acid (anhydrous) M", Fuso Chemical Co., Ltd., Osaka, Japan) under stirring. Stearamidopropyl dimethylamine (Product Name "CATINAL MPAS", Toho Chemical Industry Co., Ltd., Tokyo, Japan) in an amount of 1.2 g was further added thereto and made completely dissolved. The solution was cooled to 70°C and then added with 1.84 g of cetanol (Product Name "Cetanol", KOKYU ALCOHOL KOGYO CO., LTD. Chiba, Japan) and 2.96 g of stearyl alcohol (Product Name "Deodorized stearyl alcohol", KOKYU ALCOHOL KOGYO CO., LTD. Chiba, Japan), followed by stirring for 30 mins. Thereafter, the solution was further cooled to 65°C and stirred for further 30 mins so that it was emulsified and thickened. The solution was cooled to room temperature while stirring, added with 0.4 g of phenoxyethanol (Product Name "Hisolv EPH", Toho Chemical Industry Co., Ltd., Tokyo, Japan), and further added with the isomaltooligosaccharide-containing saccharide (Product Name "Lissenare" (Registered Trademark)) (solid content of 74% by mass or more; isomaltooligosaccharide content per solid of 50% by mass or more; and panose content per solid of 28% by mass or more) as an isomaltooligosaccharide in an amount of 0.13, 0.67, 1.33, 6.77, 13.33, 26.67, 33.33, or 40.00 g to obtain Test Emulsion Compositions 1 to 8. Control Emulsion Composition was prepared in the same manner as in Test Emulsion Compositions 1 to 8 except that ultrapure water was used instead of the isomaltooligosaccharide-containing saccharide. The compositions of Test Emulsion Compositions 1 to 8 and Control Composition were shown in Table 17.

[Table 17]

| | Control Emulsion Composition | Test Emulsion Composition | | | | | | | |
|---|---|---|---|---|---|---|---|---|---|
| | | 1 | 2 | 3 | 4 | 5 | 6 | 7 | 8 |
| Isomaltooligosaccharide-containing saccharide | - | 0.13 g | 0.67 g | 1.33 g | 6.67 g | 13.33 g | 26.67 g | 33.33 g | 40 g |
| Ultrapure water | 45 g | 45 g | 45 g | 45 g | 45 g | 45 g | 45 g | 45 g | 45 g |
| Citric acid | 0.68 g | 0.68 g | 0.68 g | 0.68 g | 0.68 g | 0.68 g | 0.68 g | 0.68 g | 0.68 g |
| Stearamidopropyl dimethylamine | 1.2 g | 1.2 g | 1.2 g | 1.2 g | 1.2 g | 1.2 g | 1.2 g | 1.2 g | 1.2 g |
| Cetanol | 1.84 g | 1.84 g | 1.84 g | 1.84 g | 1.84 g | 1.84 g | 1.84 g | 1.84 g | 1.84 g |
| Stearyl alcohol | 2.96 g | 2.96 g | 2.96 g | 2.96 g | 2.96 g | 2.96 g | 2.96 g | 2.96 g | 2.96 g |
| Phenoxyethanol | 0.4 g | 0.4 g | 0.4 g | 0.4 g | 0.4 g | 0.4 g | 0.4 g | 0.4 g | 0.4 g |
| Ultrapure water (Later Addition) | Bal. | Bal. | Bal. | Bal. | Bal. | Bal. | Bal. | Bal. | Bal. |
| Total (g) | 100 g | 100 g | 100 g | 100 g | 100 g | 100 g | 100 g | 100 g | 100 g |

<Experiment 7-2: Evaluation of Feeling of Use and Hair Treatment Effect>

The feeling of use and the hair treatment effect were evaluated at 4 stages during the process of from applying Test Emulsion Compositions 1 to 8 and Control Emulsion Composition prepared in Experiment 7-1 to the damaged hair prepared in Experiment 6-2, rinsing the hairs with tap water to wash off the applied emulsion compositions, towel drying, to drying the hair, i.e., "during application", "during rinsing", "after towel-drying", and "after drying". The feeling of use and the hair treatment effect were evaluated by 3 trained panelists by touching hairs with bare hands.

The detailed procedure was shown below. Firstly, to 4 g of the damaged hair prepared in Experiment 6-2, 1 mL of an aqueous solution containing sodium laureth sulfate (prepared by diluting sodium laureth sulfate (Product Name "EMAL E-27C", Kao Corporation, Tokyo Japan) 10-fold with ultrapure water) was put and the hair was washed with lathering for 30 seconds. The hair was rinsed under running tap water for 30 seconds and towel-dried. Thereafter, Test Emulsion Compositions 1 to 8 prepared in Experiment 7-1 were applied from the root to the tip of the damaged hair. The spreadability of each Test Emulsified Composition on the hair during application was evaluated as an indicator of the feeling of use, which was used as the evaluation at a step of "during application". Thereafter, the hair was rinsed under running tap water for 30 seconds. During rinsing, finger combability of the damaged hair was evaluated as an indicator of the feeling of use, and smoothness of the hair was evaluated as an indicator of the hair treatment effect, which were used as the evaluations at a step of "during rinsing". The damaged hair was towel-dried, air-dried with a dryer for 2 minutes, and then the moistness, smoothness and silkiness of the damaged hair were evaluated as indicators of the hair treatment effect, which were used as the evaluation at a step of "after towel-drying". After further incubating the damaged hair in an environment with a temperature of 22 to 26 °C and a relative humidity of 50 to 60% for 2 to 3 hours, the moistness, suppleness, smoothness silkiness, and luster of the damaged hair were evaluated as indicators of the hair treatment effect, which were used as the evaluation at a step of "after drying"

The "spreadability to hair" during application refers to a spreadability of the emulsion composition when applied to hair. In this experiment, the spreadability of each emulsion composition when applied to hair was evaluated, using the spreadability of Control Emulsion Composition when applied to the damaged hair as a standard for evaluation. The "finger combability" during rinsing refers to the degree of hair entanglement during rinsing. In this experiment, the degree of hair entanglement during rinsing was evaluated, using the damaged hair applied with Control Emulsion Composition as a standard for evaluation. The "smoothness" during rinsing refers to the absence of resistance feeling during rinsing. In this experiment, the degree of slipperiness of hair during rinsing was evaluated using the damaged hair applied with Control Emulsion Composition as a standard for evaluation. The "moistness" after towel-drying refers to the moisturized feeling when touching hair. In this experiment, the moisturized feeling when touching hair was evaluated using the damaged hair applied with Control Emulsion Composition as a standard for evaluation. The "smoothness" after towel-drying refers to the absence of resistance feeling. In this experiment, the degree of slipperiness of hair was evaluated using the damaged hair applied with Control Emulsion Composition as a standard for evaluation. The "silkiness" after towel-drying refers to the ease of being detangled without being entangled when touching hair. In this experiment, the ease of finger combability when touching hair was evaluated using the damaged hair applied with Control Emulsion Composition as a standard for evaluation. The "moistness" after drying refers to the moisturized feeling when touching hair. In this experiment, the moisturized feeling when touching hair was evaluated using the damaged hair applied with Control Emulsion Composition as a standard for evaluation. The "suppleness" after drying refers to the degree of flexibility that allows hair to bend without stiff feeling when being touched. In this experiment, the degree of flexibility that allows the hair to bend without stiff feeling when being touched was evaluated using the damaged hair applied with Control Emulsion Composition as a standard for evaluation. The "smoothness" after drying refers to the absence of resistance feeling. In this experiment, the degree of slipperiness of hair was evaluated using the damaged hair applied with Control Emulsion Composition as a standard for evaluation. The "silkiness" after drying refers to the ease of being detangled without being entangled when touching hair. In this experiment, the ease of finger combability when touching hair was evaluated using the damaged hair applied with Control Emulsion Composition as a standard for evaluation. As to "Luster", the degree of luster seen when hair bundles were aligned was visually evaluated, using the damaged hair applied with Control Emulsion Composition as a standard for evaluation.

Evaluation for each item was carried out on a 9-point scale based on the criteria of "4: very good", "3: good", "2: slightly good", "1: rather good", "0: not changed", "-1: rather bad", "-2: slightly bad", "-3: bad", "-4: very bad", compared to damaged hair applied with Control Emulsion Composition. The average point was calculated by dividing the sum of the points of all panelists by the number of panelists. It was judged "◎" when the average point was more than 2 points but 4 points or less, "○" when the average point was more than 0 point but 2 points or less, "△" when the average point was more than -2 points but 0 point or less, and "×" when the average point was -2 points or less. The obtained results were shown in Table 18.

[Table 18]

| | Isomaltooligosaccha-ride Content (in terms of solid content) | During application | During rinsing | | After towel-drying | | | After drying | | | | | Overall |
|---|---|---|---|---|---|---|---|---|---|---|---|---|---|
| | | Spreadability to hair | Finger Combability | Smoothness | Moistness | Smoothness | Silkiness | Moistness | Suppleness | Smoothness | Silkiness | Luster | |
| Control Emulsion Composition | 0% by mass | - | - | - | - | - | - | - | - | - | - | - | - |
| Test Emulsion Composition 1 | 0.1% by mass | ○ | △ | ○ | △ | ○ | △ | △ | △- | ○ | ○ | △ | ○ |
| Test Emulsion Composition 2 | 0.5% by mass | ○ | ○ | ○ | ○ | ○ | ○ | ○ | ○ | ○ | ○ | △ | ○ |
| Test Emulsion Composition 3 | 1% by mass | ○ | ○ | ○ | ○ | ○ | ○ | ○ | ○ | ○ | ○ | ○ | ○ |
| Test Emulsion Composition 4 | 5% by mass | ○ | ○ | ◎ | ○ | ○ | ◎ | ○ | ○ | ◎ | ○ | ○ | ○ |
| Test Emulsion Composition 5 | 10% by mass | ◎ | ○ | ◎ | ○ | ○ | ◎ | ○ | ○ | ◎ | ○ | ○ | ○ |
| Test Emulsion Composition 6 | 20% by mass | ○ | ○ | ◎ | ◎ | ○ | ○ | ○ | ○ | ○ | ○ | ○ | ○ |
| Test Emulsion Composition 7 | 25% by mass | ○ | ○ | ○ | ○ | ○ | ○ | ○ | ○ | ○ | ○ | ○ | ○ |
| Test Emulsion Composition 8 | 30% by mass | △ | ○ | ○ | ○ | ○ | △ | ○ | △ | △ | △ | ○ | ○ |

As a result of testing with the damaged hairs applied with Test Emulsion Compositions 1 to 8, which were prepared to have different final concentration of isomaltooligosaccharide in the range of 0.1 to 30% by mass using the isomaltooligosaccharide mixture prepared by mixing isomaltose and panose at the mass ratio of 1:3, as an isomaltooligosaccharide, the following results were obtained for each step of "during application", "during rinsing", "after towel-drying" and "after drying".

"During Application"

Dyring application, the "spreadability to hair" was evaluated as an indicator for the feeling of use. The "spreadability to hair" was judged favorable (i.e., judged "○" or "◎") when the final concentration of isomaltooligosaccharide in Test Emulsion Compositions was in the range of 0.1 to 25% by mass, and judged very favorable (i.e., judged "◎") when the final concentration of isomaltooligosaccharide was 10% by mass.

"During Rinsing"

During rinsing, the "finger combability" and the "smoothness" were evaluated as indicators for the feeling of use and the treatment effect, respectively. The "finger combability", an indicator for the feeling of use, was judged favorable (i.e., judged "o") when the final concentration of isomaltooligosaccharide in Test Emulsion Compositions was in the range of 0.5 to 30% by mass. Meanwhile, the "smoothness", an indicator for the treatment effect, was judged favorable (i.e., judged "○" or "◎") when the final concentration of isomaltooligosaccharide in Test Emulsion Compositions was in the range of 0.1 to 30% by mass, and very favorable (i.e., judged " ◎ ") when the final concentration of isomaltooligosaccharide in Test Emulsion Compositions was in the range of 5 to 20% by mass.

"After Towel-Drying"

After towel-drying, the "moistness", "smoothness" and "silkiness" were evaluated as indicators for the treatment effect. The "moistness" was judged favorable (i.e., judged "○" or "◎") when the final concentration of isomaltooligosaccharide in Test Emulsion Compositions was in the range of 0.5 to 30% by mass, and very favorable (i.e., judged " ◎ ") when the final concentration of isomaltooligosaccharide in Test Emulsion Compositions was 20% by mass. The "smoothness" was judged favorable (i.e., judged "o") when the final concentration of isomaltooligosaccharide in Test Emulsion Compositions was in the range of 0.1 to 30% by mass. The "silkiness" was judged favorable (i.e., judged "o" or "◎") when the final concentration of isomaltooligosaccharide in Test Emulsion Compositions was in the range of 0.5 to 30% by mass, and very favorable (i.e., judged "◎") when the final concentration of isomaltooligosaccharide in Test Emulsion Compositions was in the range of 5 to 10% by mass.

"After Drying"

After drying, the "moistness", "suppleness", "smoothness", "silkiness", and "luster" were evaluated as indicators for the treatment effect. The "moistness" was judged favorable (i.e., judged "o") when the final concentration of isomaltooligosaccharide in Test Emulsion Compositions was in the range of 0.5 to 30% by mass. The "suppleness" was judged favorable (i.e., judged "o") when the final concentration of isomaltooligosaccharide in Test Emulsion Compositions was in the range of 0.5 to 25% by mass. The "smoothness" was judged favorable (i.e., judged "○" or "◎") when the final concentration of isomaltooligosaccharide in Test Emulsion Compositions was in the range of 0.5 to 25% by mass and very favorable (i.e., judged " ◎ ") when the final concentration of isomaltooligosaccharide in Test Emulsion Compositions was in the range of 5 to 10% by mass. The "silkiness" was judged favorable (i.e., judged "o") when the final concentration of isomaltooligosaccharide in Test Emulsion Compositions was in the range of 0.1 to 25% by mass. The "luster" was judged favorable (i.e., judged "o") when the final concentration of isomaltooligosaccharide in Test Emulsion Compositions was in the range of 1 to 30% by mass.

"Overall Judgement"

Overall, it was judged favorable (i.e., judged "o") when the final concentration of isomaltooligosaccharide in Test Emulsion Compositions was in the range of 0.1 to 30% by mass.

The above result indicates that when isomaltooligosaccharide is comprised as an active ingredient, a hair-treatment composition having the good feeling of use and the treatment effect can be obtained when isomaltooligosacchairde is used so that the final concentration of isomaltooligosaccharide is in the range of 0.1 to 30% by mass with respect to the total mass of the hair-treatment composition. It was further shown that a hair-treatment composition having the particularly good feeling of use and treatment effect can be obtained preferably when the final concentration of isomaltooligosaccharide is in the range of 1 to 25% by mass, and more preferably when the final concentration of isomaltooligosaccharide is in the range of 5 to 20% by mass.

<Experiment 8: Effect of Isomaltooligosaccharide on Hair Damage>

To examine the effect of isomaltooligosaccharide on hair damage, a bending test for evaluating the softness of hairs and a hair tensile property test for evaluating the tensile strength of hairs were performed for damaged hairs treated with a solution containing an isomaltooligosaccharide. As an isomaltooligosaccharide, the isomaltooligosaccharide-containing saccharide (Product Name "Lissenare" (Registered Trademark)) (solid content of 74% by mass or more; isomaltooligosaccharide content per solid of 50% by mass or more; and panose content per solid of 28% by mass or more) was used.

<Experiment 8-1: Preparation of Test Hair Bundles (Healthy Hair, Damaged Hair, Test Sample-Treated Hair Bundle, and Control Sample-Treated Hair Bundle) for Measuring Bending Stiffness of Hairs>

The healthy hair, damaged hair, Test Sample-treated hair bundles, and Control Sample-treated hair bundles were prepared in the following method.

"Preparation of Healthy Hair"

Hair strands (Product Name "Human black hair BS-PS"; Beaulax Co., Ltd., Saitama, Japan) were aligned at the roots to a length of 20 cm to obtain a hair bundle, and 1 g of the hair bundle was washed with an aqueous solution containing 0.5% by mass of sodium laureth sulfate (prepared by diluting sodium laureth sulfate (Product Name "EMAL E-27C", Kao Corporation, Tokyo Japan) 3-fold with ultrapure water) to obtain healthy hairs.

"Preparation of Damaged Hair"

The prepared healthy hairs were washed with an aqueous solution containing 0.5% by mass of sodium laureth sulfate (prepared by diluting sodium laureth sulfate (Product Name "EMAL E-27C", Kao Corporation, Tokyo Japan) 3-fold with ultrapure water) for 3 mins, rinsed thoroughly with running tap water, and towel-dried. Then, it was immersed in a bleach solution (an aqueous solution containing equal amounts of hydrogen peroxide solution (4.0% by mass in water) and ammonia solution (2.5% by mass in water)) for 30 minutes, thoroughly rinsed with tap water, and towel-dried. Thereafter, the hair bundle was immersed in a solution of the first permanent wave agent (An aqueous solution obtained by mixing 13.0% by mass of 50% ammonium thioglycolate solution, 3.0% by mass of 28% ammonia solution, and 84.0% by mass of ultrapure water) for 15 mins, rinsed thoroughly with warm tap water, and towel-dried. The hair bundle was then immersed in the second permanent wave agent (an aqueous solution of sodium bromate (6.0% by mass in water)) for 15 minutes, thoroughly rinsed with warm tap water, and towel dried. Then, using an ultrasonic cleaner (Product Name "Bransonic 1510", Yamato Scientific Co., Ltd., Tokyo, Japan) filled with ultrapure water, the hair bundle was washed for 10 minutes at 42 kHz, and then towel-dried. Damaged hairs were obtained by repeatedly subjecting the healthy hairs to a series of treatments from washing with the aqueous solution containing 0.5% by mass of sodium laureth sulfate, washing with the ultrasonic cleaner, to towel-drying, for three times and thereafter by rinsing with ultrapure water and drying naturally.

"Preparation of Test Sample-Treated Hair Bundle and Control Sample-Treated Hair Bundle"

A hair bundle consisting of 100 g of the thus prepared damaged hairs was immersed in an aqueous solution containing 5% by mass of the isomaltooligosaccharide-containing saccharide (Product Name "Lissenare" (Registered Trademark); Hayashibara Co., Ltd., Okayama, Japan) for 10 mins, rinsed with ultrapure water, dried naturally, and then incubated for 3 hours in an environment with a temperature of 20°C±5 °C and a relative humidity of 64± 10% to obtain a Test Sample-treated hair bundle. Meanwhile, as a control, a Control Sample-treated hair bundle was obtained in the same method as in the Test Sample-treated hair bundle except that an aqueous solution containing 5% by mass of sorbitol (Product Name "SORBITOL KAO", Kao Corporation, Tokyo, Japan), which are conventionally used for the purpose of moisturizing and treating hair, was used instead of the aqueous solution containing 5% by mass of the isomaltooligosaccharide-containing saccharide.

<Experiment 8-2: Measurement of Bending Stiffness>

The softness of the hair was evaluated based on the bending stiffness of Test Sample-applied hair bundles.

The bending stiffness of the hair bundle was measured using a bending tester (Product Name "KES-FB2-S-DC", Kato Tech Co., Ltd., Kyoto, Japan). Specifically, fifty hairs were taken out from each of the healthy hairs, damaged hairs, Test Sample-treated hair bundle, and Control Sample-treated hair bundle, their roots were aligned, and they were spread within a width of 5 cm. The hairs were pasted on a mount paper at both ends with a 1 cm gap in between. Using these hairs pasted on the mount paper, the bending stiffness when the bending tester bended the hair bundle was measured, in a room conditioned to a temperature of 23.0 to 23.6°C and a relative humidity of 49 to 53%. For measurement, the sensitivity was set to full scale 20g, and bending curvature was set to 1.0 cm$^{-1}$. The obtained bending stiffness was used as an indicator of the hair softness. Measurements of the bending stiffness were performed three times for each of Test Sample-treated hair bundle, and Control Sample-treated hair bundle, the healthy hair, and the damaged hair using three similarly prepared hair bundles. Table 19 shows mean and standard deviation of the bending stiffness which was measured for each of Test Sample-treated hair bundle, and Control Sample-treated hair bundle, the healthy hair, and the damaged hair.

<Statistical Analysis>

Measured values were expressed as mean ± standard deviation (SD). Statistical analysis was performed by unpaired t-test. A significance level (p value) of less than 0.05 (less than 5% risk) was judged to be significant.

[Table 19]

|  | Bending Stiffness (B Value) (gf· cm$^2$/cm) |
|---|---|
| Healthy Hair | 0.146±0.001 |
| Damaged Hair | 0.171±0.002 |
| Hair Bundle Treated with Test Composition | 0.154±0.001** |
| Hair Bundle Treated with Control Composition | 0.169±0.002 |
| ** $p < 0.01$ vs Damaged Hair | |

As shown in Table 19, the bending stiffness (B value) of the healthy hair was 0.146±0.001 (gf· cm$^2$/cm), whereas that of the damaged hair was 0.171 ± 0.002 (gf· cm$^2$/cm), indicating the increased bending stiffness in the damaged hair. On the other hand, the bending stiffness of Control Sample-treated hair bundle prepared by treating the damaged hair with the aqueous solution containing sorbitol, which was conventionally used for the purpose of moisturizing and hair treatment, was 0.169±0.002 (gf· cm$^2$/cm) and was comparable to that of the damaged hair. In contrast, the bending stiffness of Test Sample-treated hair bundle prepared by treating the damaged hair with the aqueous solution containing an isomaltooligosaccharide was 0.154±0.001 (gf· cm$^2$/cm) and was remarkably smaller than that of the damaged hair. This result indicates that an isomaltooligosaccharide has the effect of decreasing the stiffness of hair that is increased due to hair damages and that this effect is superior to that of sorbitol conventionally used for the purpose of moisturizing and hair treatment.

<Experiment 8-3: Preparation of Test Hair Bundles (Healthy Hair, Damaged Hair, Test Sample-Treated Hair Bundle, and Control Sample-Treated Hair Bundle) for Measuring Tensile Strength of Hair>

The healthy hair, damaged hair, Test Sample-treated hair bundle, and Control Sample-treated hair bundle were prepared in the following method.

"Healthy Hair"

Hair strands (Product Name "Human black hair BS-PS"; Beaulax Co., Ltd., Saitama, Japan) were aligned at the roots to a length of 30 cm to obtain a hair bundle, and 7 g of the hair bundle was immersed in an aqueous solution containing 1% by mass of sodium laureth sulfate (prepared by diluting sodium laureth sulfate (Product Name "EMAL E-27C", Kao Corporation, Tokyo Japan) 3-fold with ultrapure water) for 1 min, rinsed with tap water for 1 min, drained with paper clothes, and dried with a dryer to obtain healthy hairs.

"Damaged Hair"

The prepared healthy hairs were immersed in a bleaching solution (aqueous solution obtained by mixing equal volume of 3% by mass of hydrogen peroxide solution and 3% by mass of ammonia solution) for 20 mins, rinsed with tap water for 1 min, drained with paper clothes, and dried with a dryer. Damaged hairs were obtained by repeatedly subjecting the healthy hairs to a series of the above treatments from immersion in the bleaching solution to drying using a dryer, for three times.

"Test Sample-Treated Hair Bundle and Control Sample-Treated Hair Bundle"

Hundred (100) grams of a hair bundle of the damaged hair was immersed in an aqueous solution containing 5% by mass of an isomaltooligosaccharide-containing saccharide (Product Name "Lissenare" (Registered Trademark); Solid Content 75%; Hayashibara Co., Ltd., Okayama, Japan) for 10 mins, rinsed with ultrapure water for 10 seconds, drained with paper clothes, and dried using a dryer to obtain a Test Sample-Treated Hair Bundle. Meanwhile, as a control, a Control Sample-treated hair bundle was obtained in the same method as in the preparation of the Test Sample-treated hair bundle except that an aqueous solution containing 5% by mass of sorbitol (Product Name "SORBITOL KAO", Kao Corporation, Tokyo, Japan), which are conventionally used for the purpose of moisturizing and hair treatment, was used instead of the aqueous solution containing 5% by mass of the isomaltooligosaccharide-containing saccharide (Product Name "Lissenare" (Registered Trademark); Solid Content 75%; Hayashibara Co., Ltd., Okayama, Japan).

<Experiment 8-4: Measurement of Tensile Strength of Hairs>

The tensile strength of hair was evaluated by pulling Test Sample-treated hair and using the load applied to the hair until the hair was broken as an indicator. The load was measured using an autograph (Shimadzu Corporation, Kyoto, Japan). Specifically, the Test Sample-treated hair bundle, Control Sample-treated hair bundle, healthy hair, and damaged hair were conditioned at room temperature ($20\pm5°C$; relative humidity $64\pm10\%$) for 24 hours or more. One hair was taken from each of the Test Sample-treated hair bundle, Control Sample-treated hair bundle, healthy hair, and damaged hair and the root side of the hair was fixed to the upper chuck of the autograph. A weight of 0.005 N was used to remove the flexure of the hair, and the tip side of the hair was fixed to the lower chuck of the autograph. The hair was pulled at a speed of 60 mm/min, and the load when the hair was broken was measured and used as an indicator of the tensile strength of the hair. Measurements of the load were performed five times for each of the healthy hair, damaged hair, Test Sample-treated hair bundle, and Control Sample-treated hair bundle using five hairs taken from each sample. Table 20 shows mean and standard deviation of the load when the hair was broken which was measured for each of Test Sample-treated hair bundle, and Control Sample-treated hair bundle, the healthy hair, and the damaged hair.

<Statistical Analysis>

Measured values were expressed as mean $\pm$ standard deviation (SD). Statistical analysis was performed by unpaired t-test. A significance level (p value) of less than 0.05 (less than 5% risk) was judged to be significant.

[Table 20]

|  | Load (N) |
|---|---|
| Healthy Hair | $0.915\pm0.029$ |
| Damaged Hair | $0.876\pm0.056$ |
| Hair Bundle Treated with Test Composition | $1.065\pm0.070$** |
| Hair Bundle Treated with Control Composition | $0.942\pm0.154$ |
| ** p < 0.01 vs Damaged Hair | |

As shown in Table 20, the load applied to the hair when the hair was pulled and broken was $0.915\pm0.029$N for the healthy hair, whereas that for the damaged hair was $0.876\pm0.056$N, indicating that the tensile strength was decreased, in other words, the hair became brittle in the damaged hair. On the other hand, for the Control Sample-treated hair bundle prepared by treating the damaged hair with the aqueous solution containing sorbitol, the load applied to the hair when the hair was pulled and broken was $0.942\pm0.154$N, which was larger than that for the damaged hair but the difference was not statistically significant (p>0.05 vs the damaged hair). In contrast, for the Test Sample-treated hair bundle prepared by treating the damaged hair with the aqueous solution containing an isomaltooligosaccharide, the load applied to the hair when the hair was pulled and broken was $1.065\pm0.070$N, which was remarkably larger than that measured for the damaged hair. This result indicates that an isomaltooligosaccharide has the effect of improving a strength of hair that becomes brittle due to hair damages, and further that this effect is superior to that of sorbitol, which is conventionally used for the purpose of moisturizing and treating hair. It was suggested that a composition comprising an isomaltooligosaccharide has the effect of preventing the occurrence of split ends and cut hairs due to hair damages.

The above results indicate that an isomaltooligosaccharide not only enhances the hair-setting ability when applied to hair, but also improves the softness and the strength of hair after being washed out and shows the hair treatment effect for protecting hair.

<Experiment 9: Effect of Isomaltooligosaccharide on Denaturation of Proteins by Surfactants>

In order to examine the effect of surfactants on denaturation of proteins and the effect of an isomaltooligosaccharide on it, the rate of dissolution of zein, an insoluble protein, was evaluated as an indicator. A zein test is a method to evaluate the occurrence of rough skin using a denaturation (dissolution) of zein, an insoluble protein, by surfactants as an indicator.

<Experiment 9-1: Preparation of Test Sample and Control Sample>

"Preparation of Test SDS Solution"

An isomaltooligosaccharide solution was obtained by using the isomaltooligosaccharide-containing saccharide (Product Name "Lissenare" (Registered Trademark); Solid Content 75%; Hayashibara Co., Ltd.) as an isomaltooligosaccharide and by adding 10 g of ultrapure water to 13.3 g of the isomaltooligosaccharide-containing saccharide and uniformly mixing the mixture using a stirrer. To the obtained isomaltooligosaccharide solution, 5 g of an aqueous solution containing 10% by mass of sodium dodecyl sulfate (prepared by diluting sodium dodecyl sulfate (Product Name "EMAL 10", Kao Corporation, Tokyo Japan) 3-fold with ultrapure water) was added. Ultrapure water was further added thereto so that the total mass was 50 g. After 15 mins of stirring, a test sodium dodecyl sulfate solution (hereinafter referred to as "Test SDS Solution") containing an isomaltooligosaccharide was obtained. The isomaltooligosaccharide content and the sodium dodecyl sulfate content of Test SDS solution were 20% by mass and 1% by mass, respectively.

"Preparation of Control SDS Solution"

Meanwhile, a control sodium dodecyl sulfate solution (hereinafter referred to as "Control SDS Solution") was obtained by mixing 45 g of ultrapure water and 5 g of an aqueous solution containing 10% by mass of sodium dodecyl sulfate (prepared by diluting sodium dodecyl sulfate (Product Name "EMAL 10", Kao Corporation, Tokyo Japan) 3-fold with ultrapure water). The sodium dodecyl sulfate content of Control SDS solution was 1% by mass, which was the same as in Test SDS solution.

<Experiment 9-2: Measurement of Zein Dissolution Rate>

The weight of the beaker used for the measurement with the stirrer in it ("the weight of the beaker containing the stirrer"), the weight of the filter paper, and the weight of the weighing dish were weighed.

Next, 2 g of zein (Product Name "Zein (for biochemistry)", FUJIFILM Wako Pure Chemical Corporation, Osaka, Japan) was precisely weighed on the weighing dish, which was added to 50 g of Test SDS Solution or Control SDS Solution under stirring and suspended. The mixture was stirred for 60 minutes and allowed to stand for 5 minutes or more, followed by suction filtration using a Buchner funnel with the filter paper (Product Name "Whatman filter paper No. 541 7 cm$\phi$", Whatman plc., United Kingdom). The filter paper and the stirrer used for stirring were placed in a water-washed beaker and dried in an incubator at 45°C for 24 hours. The weight of the beaker containing the filter paper and the stirrer ("the weight of the beaker containing the filter paper and the stirrer after drying for 24 hours") was weighed. And, the weight of the weighing dish, on which zein was weighed, after adding the weighed zein to the SDS solutions ("the weight of the weighing dish after adding zein") was weighed. The amount of added zein was calculated as "the weight of the weighing dish" + "the weight of zein of 2g" - "the weight of the weighing dish after unloading zein". Meanwhile, the amount of remaining zein was calculated as "the weight of the beaker containing the filter paper and the stirrer after drying for 24 hours" - "the weight of the beaker containing the stirrer" - "the weight of the filter paper". Using these values, the zein dissolution rates were obtained following the below formula 1. The zein dissolution rates of Test SDS Solution and Control SDS Solution were measured three times using three independently prepared solutions for each sample. Mean and standard deviation of the thus obtained zein dissolution ratio were shown in Table 21.

Formula 1

$$\text{Zein Dissolution Rate (\%)} = [(\text{the amount of added zein}) - (\text{the amount of remaining zein})]/(\text{the amount of added zein}) \times 100$$

<Statistical Analysis>

Measured values were expressed as mean $\pm$ standard deviation (SD). Statistical analysis was performed by unpaired t-test. A significance level (p value) of less than 0.05 (less than 5% risk) was judged to be significant.

[Table 21]

|  | Zein Dissolution Rate (%) |
|---|---|
| Control SDS Solution | 91.34 $\pm$ 0.66 |
| Test SDS Solution | 78.64 $\pm$ 1.51*** |
| ** : p<0.01 vs Control SDS Solution | |

As shown in Table 21, the measured zein dissolution rate was $91.34\pm0.66\%$ for Control SDS Solution not containing isomaltooligosaccharides. Meanwhile, the measured zein dissolution rate was $78.64\pm1.51\%$ for Test SDS Solution containing isomaltooligosaccharides, which was revealed to be significantly lower than that measured for Control SDS Solution. This result suggests that isomaltooligosaccharides are useful for reducing occurrence of rough skin caused by surfactants and preventing rough skin on the scalp and the like.

The present invention will be described in more detail below with reference to examples. That is, examples of the agent for enhancing a hair-setting ability will be described in Examples 1 to 5, examples of the hair-setting composition comprising the agent for enhancing a hair-setting ability will be described in Examples 6 to 19. However, the scope of the present invention is not limited by these examples.

**Example 1**

<Agent for Enhancing Hair-Setting Ability>

An isomaltooligosaccharide-containing saccharide powder was prepared by spray-drying the isomaltooligosaccharide-containing saccharide solution prepared in Experiment 1-1 in accordance with the conventional method. This product can be used as an agent for enhancing a hair-setting ability of hair-setting compositions.

**Example 2**

In accordance with the method described in Example 1 of Japanese Patent Application Publication No. H4-360663, an isomaltooligosaccharide-containing saccharide solution was prepared. Briefly, a maltose-rich syrup (Product Name "MALTRUP", Hayashibara Co., Ltd., Okayama, Japan) was adjusted to a concentration of 35% by mass, to which 300 units (per 1 g of substrate) of transglucosidase derived from *Aspergillus niger* (Amano Enzyme Inc., Aichi, Japan) were added. The mixture was allowed to react at pH 5.5 and at 55°C for 24 hours, followed by heat-inactivation of the enzyme. In accordance with a conventional method, the obtained solution was purified through filtration, decoloration, and desalting using ion-exchange resins (H type and OH type), and then concentrated to obtain an isomaltooligosaccharide-containing saccharide solution. As a result of analysis in accordance with a conventional method, the saccharide composition of this solution was determined to be 24.3% by mass of glucose, 14.2% by mass of maltose, 10.5% by mass of isomaltose, 0% by mass of maltotriose, 33.8% by mass of panose, 0.6% by mass of isomaltotriose, and 15.6% by mass of tetrasaccharide or higher oligosaccharides. This product can be used as an agent for enhancing a hair-setting ability of hair-setting compositions.

### Example 3

<Agent for Enhancing Hair-Setting Ability>

An isomaltooligosaccharide-containing amorphous powder was prepared by spray-drying the isomaltooligosaccharide-containing saccharide solution prepared in Experiment 1-1 in accordance with the conventional method. This product can be used as an agent for enhancing a hair-setting ability of hair-setting compositions.

### Example 4

In accordance with the method described in Example 2 of Japanese Patent Application Publication No. H4-360663, an isomaltooligosaccharide-containing saccharide solution was prepared. Briefly, cornstarch was used as a raw material and liquefied in accordance with a conventional method to obtain a liquefied liquid having a concentration of 35% and a glucose equivalent (DE) of 12. To this liquefied liquid, 6.4 units of β-amylase derived from soybean (Nagase & Co., Ltd, Tokyo Japan) and 300 units (per 1g of solid starch) of transglucosidase derived from *Aspergillus niger* (Amano Enzyme Inc., Aichi, Japan) were added. The mixture was allowed to react at pH 5.5 and at 55°C for 12 hours, followed by heat-inactivation of the enzyme. In accordance with a conventional method, the obtained solution was purified through filtration, decoloration, and desalting using ion-exchange resins (H type and OH type), and then concentrated to obtain an isomaltooligosaccharide-containing saccharide solution. As a result of analysis in accordance with a conventional method, the saccharide composition of this solution was determined to be 14.4% by mass of glucose, 10.7% by mass of maltose, 7.1% by mass of isomaltose, 2.0% by mass of maltotriose, 21.3% by mass of panose, 0% by mass of isomaltotriose, and 43.5% by mass of tetrasaccharide or higher oligosaccharides. This product can be used as an agent for enhancing a hair-setting ability of hair-setting compositions.

### Example 5

<Agent for Enhancing Hair-Setting Ability>

An isomaltooligosaccharide-containing amorphous powder was prepared by spray-drying the isomaltooligosaccharide-containing saccharide solution prepared in the method of Example 4 in accordance with the conventional method. This product can be used as an agent for enhancing a hair-setting ability of hair-setting compositions.

### Example 6

<Setting Lotion>

| | |
|---|---|
| Isomaltose (in terms of solid) | 1 part by mass |
| Panose | 3 parts by mass |
| Isomaltooligosaccharide-containing saccharide (Product Name "Lissenare" (Registered Trademark), Hayashibara Co., Ltd., Okayama, Japan) (in terms of solid) | 1 part by mass |
| Ethanol | 10 parts by mass |
| Silicone derivative | 0.5 parts by mass |
| 1,3-Butylene glycol | 2 parts by mass |
| Phenoxyethanol | 0.4 parts by mass |

Adding refined water to make a total mass 100 parts by mass.
A setting lotion was prepared by stirring and dissolving the above components in a conventional manner. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 7**

<Setting Lotion>

| | |
|---|---|
| The isomaltooligosaccharide-containing saccharide solution prepared in the method of Experiment 1-1 (in terms of solid) | 20 parts by mass |
| Ethanol | 10 parts by mass |
| Silicone derivative | 0.5 parts by mass |
| Glycerin | 2 parts by mass |
| Parabene | 0.1 parts by mass |

Adding refined water to make a total mass 100 parts by mass.

A setting lotion was prepared by stirring and dissolving the above components in a conventional manner. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 8**

<Hair Gel>

| | |
|---|---|
| Carboxyvinyl polymer | 0.7 parts by mass |
| Polyvinylpyrrolidone | 2 parts by mass |
| Glycerin | 0.1 parts by mass |
| The isomaltooligosaccharide-containing saccharide powder prepared in the method of Example 1 | 5 parts by mass |
| Sodium hydroxide | q.s. |
| Ethyl alcohol | 15 parts by mass |
| Polyoxyethylene octyldodecyl ether | 0.2 parts by mass |
| Fragrance, Chelating agent | q.s. |

Adding refined water to make a total mass 100 parts by mass.

A hair gel was prepared by stirring and dissolving the above components in a conventional manner. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 9**

<Hair Gel>

| | |
|---|---|
| Carboxyvinyl polymer | 0.7 parts by mass |

(continued)

| | |
|---|---|
| Polyvinylpyrrolidone | 2 parts by mass |
| 1 ,3-Butylene glycol | 0.1 parts by mass |
| The isomaltooligosaccharide-containing saccharide solution prepared in the method of Example 2 (in terms of solid) | 1 part by mass |
| Trehalose (Product Name "TREHA" (Registered Trademark), Hayashibara Co., Ltd., Okayama, Japan) | 1 part by mass |
| Potassium hydroxide | q.s. |
| Ethyl alcohol | 20 parts by mass |
| Polyoxyethylene octyldodecyl ether | 0.2 parts by mass |
| Fragrance, Chelating agent | q.s. |

Adding refined water to make a total mass 100 parts by mass.

A hair gel was prepared by stirring and dissolving the above components in a conventional manner. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 10**

<Hair Liquid>

| | |
|---|---|
| Polyoxypropylene (40) butyl ether | 20 parts by mass |
| Polyoxyethylene hydrogenated castor oil | 1 parts by mass |
| Ethyl alcohol | 50 parts by mass |
| The isomaltooligosaccharide-containing amorphous powder prepared in the method of Example 3 | 0.5 parts by mass |
| Hydrogenated starch hydrolysate (Product Name Trademark), Hayashibara Co., Ltd., Okayama, Japan) "Tornare" (Registered(in terms of solid) | 0.5 parts by mass |
| Fragrance | q.s. |
| Dye | q.s. |
| Preservative | q.s. |

Adding refined water to make a total mass 100 parts by mass.

A hair liquid was prepared by stirring and dissolving the above components in a conventional manner. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 11**

<Hair Treatment Lotion>

| | |
|---|---|
| 1 ,3-Butylene glycol | 2 parts by mass |
| Glycerin | 1 part by mass |

(continued)

| | |
|---|---|
| Stearyl methyl ammonium chloride | 0.5 parts by mass |
| Methyl phenyl polysiloxane | 1 part by mass |
| Collagen hydrolysate | 1 part by mass |
| Sodium chondroitin sulfate | 0.5 parts by mass |
| The isomaltooligosaccharide-containing saccharide solution prepared in the method of Example 4 (in terms of solid) | 10 parts by mass |
| Fragrance | q.s. |
| UV absorbent | q.s. |
| Preservative | q.s. |
| Ethyl ether | 50 parts by mass |

Adding refined water to make a total mass 100 parts by mass.

A treatment lotion was prepared by stirring and dissolving the above components in a conventional manner. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 12**

<Hair Cream>

<Oil Phase>

| | |
|---|---|
| Liquid paraffin | 15 parts by mass |
| Petroleum jelly | 15 parts by mass |
| White beeswax | 2 parts by mass |
| Preservative | q.s. |
| Fragrance | q.s. |

<Water Phase>

| | |
|---|---|
| Refined water | 57.8 parts by mass |
| The isomaltooligosaccharide-containing amorphous powder prepared in the method of Example 5 | 2 parts by mass |
| Trehalose (Product Name "TREHA" (Registered Trademark), Hayashibara Co., Ltd., Okayama, Japan) | 1 part by mass |
| Carboxyvinyl polymer | 0.1 parts by mass |
| Xanthan gum | 0.1 parts by mass |
| Glycerin | 4 parts by mass |
| Polyoxyethylene hydrogenated castor oil | 3 parts by mass |
| Chelating agent | q.s. |

(continued)

| Dye | q.s. |
|---|---|

The above oil phase components were mixed and dissolved under heat. Its temperature was adjusted to 80°C and therein the above water phase components dissolved under heat and adjusted to 80°C were added under stirring, followed by emulsification using a homogenizer. After being cooled to 30°C, 0.05 parts by mass of sodium hydroxide was added and the mixture was stirred until it became uniform. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 13**

<Hair Foam>

| | |
|---|---|
| Acrylic Resin Alkanolamine Solution (50%) | 8 parts by mass |
| Polyoxyethylene hydrogenated castor oil | 0.2 parts by mass |
| Liquid paraffin | 5 parts by mass |
| Glycerin | 2 parts by mass |
| Isomaltose (in terms of solid) | 0.2 parts by mass |
| Panose | 2.0 parts by mass |
| Sodium hyaluronate | 0.5 parts by mass |
| Ethyl alcohol | 13 parts by mass |
| Fragrance, Preservative | q.s. |

Refined water was added to make a total mass 100 parts by mass, and this solution was filtered.
These components were stirred and dissolved in accordance with the conventional method and 90 parts by mass of the thus obtained solution was filled in a filling can. After attaching a valve, the filling can was further filled with 10 parts by mass of liquefied petroleum gas. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 14**

<Hair Foam>

| | |
|---|---|
| Cationized cellulose | 3 parts by mass |
| Polyoxyethylene hydrogenated castor oil | 0.2 parts by mass |
| Silicone oil | 1 part by mass |
| Dipropylene glycol | 5 part by mass |
| The isomaltooligosaccharide-containing saccharide solution prepared in the method of Experiment 1-1 (in terms of solid) | 10 parts by mass |
| Ethyl alcohol | 15 parts by mass |
| Fragrance, Preservative | q.s. |

Refined water was added to make a total mass 100 parts by mass, and this solution was filtered.

These components were stirred and dissolved in accordance with the conventional method and 90 parts by mass of the thus obtained solution was filled in a filling can. After attaching a valve, the filling can was further filled with 10 parts by mass of liquefied petroleum gas. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 15**

<Hair Spray>

| | |
|---|---|
| Acrylic Resin Alkanolamine Solution (50%) | 7 parts by mass |
| Cetyl alcohol | 0.1 parts by mass |
| Silicone oil | 0.3 parts by mass |
| The isomaltooligosaccharide-containing saccharide powder prepared in the method of Example 1 | 0.5 parts by mass |
| Hydrogenated starch hydrolysate (Product Name "Tornare" (Registered Trademark), Hayashibara Co., Ltd., Okayama, Japan) (in terms of solid) | 0.5 parts by mass |
| Ethyl alcohol | 90 parts by mass |
| Fragrance, Preservative | q.s. |

Refined water was added to make a total volume 100 parts by mass, and this solution was filtered.

This solution was filled in a filling can in accordance with the conventional method. After attaching a valve, the filling can was filled with the same amount of dimethyl ether as the solution. This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 16**

<Hair Dye>

| | |
|---|---|
| Acid dye | 1 part by mass |
| Benzyl alcohol | 6 parts by mass |
| Isopropyl alcohol | 18 parts by mass |
| The isomaltooligosaccharide-containing saccharide solution prepared in the method of Example 2 (in terms of solid) | 8 parts by mass |
| Citric acid | 0.3 parts by mass |
| Xanthan gum | 0.5 parts by mass |
| Pullulan | 0.5 parts by mass |
| Sodium chondroitin sulfate | 0.2 parts by mass |

Adding refined water to make a total mass 100 parts by mass.

This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 17**

<Hair Dye>

<First Agent>

| | |
|---|---|
| Cetostearyl alcohol | 10 parts by mass |
| Lanolin | 1 part by mass |
| p-phenylenediamine | 1 part by mass |
| Resorcinol | 1 part by mass |
| Sodium lauryl sulfate | 1 part by mass |
| The isomaltooligosaccharide-containing amorphous powder prepared in the method of Example 3 | 10 parts by mass |
| Ammonia solution (28%) | 4 parts by mass |
| Antioxidant | q.s. |
| Chelating agent | q.s. |

Adding refined water to make 100 parts by mass in total.

<Second Agent>

| | |
|---|---|
| Hydrogen peroxide solution (35%) | 17 parts by mass |
| Polyoxyethylene alkylether | 1 part by mass |
| The isomaltooligosaccharide-containing amorphous powder prepared in the method of Example 3 | 10 parts by mass |
| Cetostearyl alcohol | 3 parts by mass |
| Chelating agent | q.s. |
| pH adjusting agent | q.s |

Adding refined water to make a total mass 100 parts by mass.

This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented. Furthermore, by changing the blending ratio of p-phenylenediamine and resorcinol, or by using meta-aminophenol, para-diamine, meta-diamine or their derivatives as a coupler instead of resorcinol, this product can be optionally provided as a brown/green, brown/red-purple, blue/blue-purple, brown, and purple/blue-purple hair colorant.

**Example 18**

<Permanent Wave Agent>

<First Agent>

| | |
|---|---|
| Cysteine | 5 parts by mass |
| Ammonium Thioglycolate | 0.3 parts by mass |

(continued)

| | |
|---|---|
| Monoethanolamine | 2 parts by mass |
| The isomaltooligosaccharide-containing saccharide solution prepared in the method of Example 4 (in terms of solid) | 1 part by mass |
| Edetate | q.s. |
| Fragrance | q.s. |

Adding refined water to make a total mass 100 parts by mass.

<Second Agent>

| | |
|---|---|
| Sodium oxalate | 5 parts by mass |
| Silicone emulsion | 1 part by mass |
| The isomaltooligosaccharide-containing saccharide solution prepared in the method of Example 4 (in terms of solid) | 1 part by mass |
| pH adjusting agent | q.s. |

Adding refined water to make 100 parts by mass in total.

This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Example 19**

<Hair Growth Agent>

| | |
|---|---|
| Ethyl alcohol | 60 parts by mass |
| Hinokitiol | 0.1 parts by mass |
| Vitamin B6 | 0.2 parts by mass |
| Vitamin E derivative | 0.1 parts by mass |
| Propylene glycol | 1.5 parts by mass |
| The isomaltooligosaccharide-containing amorphous powder prepared in the method of Example 5 | 0.5 parts by mass |
| Panose | 0.5 parts by mass |
| Quaternium-51 | 0.01 parts by mass |
| Fragrance | q.s. |

Adding refined water to make a total mass 100 parts by mass.

This product has the excellent hair-setting ability and hair-arranging ability. In addition, when it is applied to hair, the hair is smooth and not sticky, the flaking is reduced, the excellent feeling of use and finish are provided, and furthermore hair softness, luster, manageability, and combability are improved and the occurrence of split ends and cut hair can be prevented.

**Industrial Applicability**

Since the agent for enhancing a hair-setting ability in accordance with one aspect of the present invention has the excellent effect of enhancing a hair-setting ability and imparting a hair-arranging ability, by formulating the agent in a hair-setting composition, a hair-setting composition that is able to hold a hairstyle may be provided. Meanwhile, the hair-setting composition in accordance with one aspect of the present invention has the smooth and not sticky feeling of use, is less prone to cause flaking, imparts excellent appearance to hair, makes hair soft, manageable and easy to comb, and prevents the occurrence of split ends and cut hair. The present invention is an invention providing such remarkable effects, and its contribution to industry is truly great and significant.

**Claims**

1. An agent for enhancing a hair-setting ability, comprising an isomaltooligosaccharide as an active ingredient.

2. The agent according to claim 1, wherein the isomaltooligosaccharide is one or more of isomaltose, panose, isomaltotriose, isomaltotetraose, isomaltopentaose, and isomaltohexaose.

3. The agent according to claim 1 or 2, which comprises isomaltose and panose as said isomaltooligosaccharide, wherein the mass ratio of isomaltose and panose in terms of their solid content is in the range of 1:1 to 1:10.

4. The agent according to any one of claims 1 to 3, having a hair-arranging ability.

5. The agent according to any one of claims 1 to 4, improving a feel of hair.

6. The agent according to claim 5, wherein said improving is reducing tangling of hair, reducing stickiness of hair, and/or enhancing smoothness of hair.

7. The agent according to any one of claims 1 to 6, suppressing an occurrence of flaking.

8. The agent according to any one of claims 1 to 7, having a hair-treatment effect.

9. The agent according to claim 8, wherein said hair-treatment effect is an enhancement of a softness of hair, an enhancement of a tensile strength of hair, and/or a reduction of a damage on a hair surface.

10. The agent according to claim 8 or 9, which is for use in protecting hair from a hair damage.

11. The agent according to any one of claims 1 to 10, reducing a rough skin of a scalp.

12. A hair-setting composition comprising the agent according to any one of claims 1 to 11.

13. The hair-setting composition according to claim 12, comprising the agent such that the total amount of the isomaltooligosaccharide is 0.1 to 30% by mass in terms of solid content.

14. The hair-setting composition according to claim 12 or 13, which is in a form of a hair water, hair mist, hair liquid, hair lotion, setting lotion, coloring lotion, hair tonic, hair gel, hair foam, hair mousse, hair spray, hair serum, hair cream, hair wax, water grease, hair oil, pomade, tique, hair stick, or hair solid.

15. The hair-setting composition according to any one of claims 12 to 14, which is in a form of a leave-on type hair-setting composition.

16. The hair-setting composition according to any one of claims 12 to 14, which is in a form of a rinse-off type hair-setting composition.

17. A method for enhancing a hair-setting ability, which comprises applying the hair-setting composition according to any one of claims 12 to 16 to hair.

Fig. 1

Fig. 2

## INTERNATIONAL SEARCH REPORT

| | International application No. |
|---|---|
| | **PCT/JP2022/008915** |

### A. CLASSIFICATION OF SUBJECT MATTER

*A61K 8/73*(2006.01)i; *A61Q 5/00*(2006.01)i; *A61Q 5/04*(2006.01)i; *A61Q 5/06*(2006.01)i
FI:  A61K8/73; A61Q5/00; A61Q5/04; A61Q5/06

According to International Patent Classification (IPC) or to both national classification and IPC

### B. FIELDS SEARCHED

Minimum documentation searched (classification system followed by classification symbols)

A61K8/73; A61Q5/00; A61Q5/04; A61Q5/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

Japio-GPG/FX

### C. DOCUMENTS CONSIDERED TO BE RELEVANT

| Category* | Citation of document, with indication, where appropriate, of the relevant passages | Relevant to claim No. |
|---|---|---|
| X | JP 2005-206483 A (KANEBO COSMETICS INC) 04 August 2005 (2005-08-04) paragraphs [0001], [0006], [0009], [0011], [0018], [0021] | 1, 4-10, 12-17 |
| Y | | 2-3, 11 |
| Y | JP 2005-89356 A (ICHIMARU PHARCOS CO LTD) 07 April 2005 (2005-04-07) paragraphs [0012], [0077], [0100], [0114] | 2-3, 11 |
| Y | JP 2005-89355 A (ICHIMARU PHARCOS CO LTD) 07 April 2005 (2005-04-07) claims, paragraphs [0001], [0007]-[0009] | 11 |
| A | JP 2013-75840 A (NAKANO SEIYAKU KK) 25 April 2013 (2013-04-25) entire text | 1-17 |
| A | JP 2008-308442 A (HOYU CO LTD) 25 December 2008 (2008-12-25) entire text | 1-17 |
| A | JP 2006-111588 A (LION CORP) 27 April 2006 (2006-04-27) entire text | 1-17 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **26 April 2022** | **17 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/008915**

| C. | DOCUMENTS CONSIDERED TO BE RELEVANT | |
|---|---|---|
| Category* | Citation of document, with indication, where appropriate, of the relevant passages | Relevant to claim No. |
| A | WO 2011/073437 A2 (L'OREAL) 23 June 2011 (2011-06-23)<br>entire text | 1-17 |

Form PCT/ISA/210 (second sheet) (January 2015)

**INTERNATIONAL SEARCH REPORT**
Information on patent family members

| International application No. |
| --- |
| **PCT/JP2022/008915** |

| Patent document<br>cited in search report | | | Publication date<br>(day/month/year) | Patent family member(s) | Publication date<br>(day/month/year) |
| --- | --- | --- | --- | --- | --- |
| JP | 2005-206483 | A | 04 August 2005 | (Family: none) | |
| JP | 2005-89356 | A | 07 April 2005 | (Family: none) | |
| JP | 2005-89355 | A | 07 April 2005 | (Family: none) | |
| JP | 2013-75840 | A | 25 April 2013 | (Family: none) | |
| JP | 2008-308442 | A | 25 December 2008 | (Family: none) | |
| JP | 2006-111588 | A | 27 April 2006 | (Family: none) | |
| WO | 2011/073437 | A2 | 23 June 2011 | FR 2954140 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002255756 A
- JP H5112437 B
- JP 2006232820 A

- JP 2009519273 W
- JP 2012236804 A
- JP H4360663 B